(19) Europäisches Patentamt
European Patent Office
Office européen des brevets

(11) EP 0 979 846 B1

(12) EUROPÄISCHE PATENTSCHRIFT

(45) Veröffentlichungstag und Bekanntmachung des Hinweises auf die Patenterteilung:
**27.02.2002 Patentblatt 2002/09**

(51) Int Cl.[7]: **C09B 5/62**, C09B 67/04

(21) Anmeldenummer: **99115365.1**

(22) Anmeldetag: **04.08.1999**

(54) **Verfahren zur Herstellung von N,N'-Dimethylperylen-3,4,9,10-tetracarbonsäurediimid in transparenter Pigmentform**

Method for the production of N,N'-dialkyl perylene-3,4,9,10-tetracarboxylic acid diimides in a transparent pigment form

Procédé pour la préparation de diimides d'acide N,N'-dialkyleperylene-3,4,9,10-tetracarboxylique dans une forme pigmentaire transparente

(84) Benannte Vertragsstaaten:
**CH DE ES FR GB IT LI**

(30) Priorität: **13.08.1998 DE 19836714**

(43) Veröffentlichungstag der Anmeldung:
**16.02.2000 Patentblatt 2000/07**

(73) Patentinhaber: **Clariant GmbH**
**65929 Frankfurt am Main (DE)**

(72) Erfinder:
- **Urban, Manfred**
  **65205 Wiesbaden (DE)**
- **Böhmer, Martin**
  **61267 Neu-Anspach (DE)**
- **Weber, Joachim, Dr.**
  **65929 Frankfurt (DE)**

(56) Entgegenhaltungen:
**EP-A- 0 318 022**
**EP-A- 0 678 559**
**EP-A- 0 864 613**
**DE-A- 2 504 481**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 979 846 B1

## Beschreibung

Gegenstand der vorliegenden Erfindung ist ein besonders umweltfreundliches und wirtschaftliches Verfahren zur Herstellung von transparenten N,N'-Dimethyl-perylen-3,4,9,10-tetracarbonsäurediimid-Pigmenten zum Pigmentieren von hochmolekularen Materialien.

Bei der Pigmentierung von Automobillacken, insbesondere den Metalliclacken, werden Pigmente benötigt, die bei hoher Transparenz und reinem Farbton farbstarke und hochglänzende Lackierungen ergeben. Die Farbkonzentrate (mill base) und die Lacke müssen niedrigviskos sein und dürfen keine Strukturviskosität zeigen. Außerdem werden sehr gute Echtheiten, insbesondere gute Wetterechtheit, verlangt.

N,N'-Dimethylperylen-3,4,9,10-tetracarbonsäurediimid (Dimethylperylimid) wird als organisches Rot- oder Marronpigment (C.I. Pigment Red 179, C.I.Nr. 71130) seit langem verwendet, wobei sowohl transparente als auch deckende Formen des Pigments bekannt sind. In Abhängigkeit von den Herstellbedingungen werden rote oder marronfarbene Pigmente erhalten. Zu seiner Herstellung werden folgende Verfahren beschrieben:

Die DE-B-21 53 087 (GB-A-1 370 433) beschreibt die Umsetzung von Perylen-3,4,9,10-tetracarbonsäuredianhydrid mit Monomethylamin bei 120 bis 150°C, wobei unter Anwendung hoher Konzentrationen des Dianhydrids gearbeitet wird. Die bei der Kondensation erhaltenen Dimethylperylimid-Rohpigmente werden isoliert, getrocknet und danach durch eine intensive Mahl- und Knetoperation mit Salz und anschließendem erneuten Trocknen in die fertige Pigmentform überführt. Nach jeder Verfahrensstufe wird das Pigment isoliert, weshalb große Mengen an Abwasser entstehen. Das Verfahren liefert jedoch kein transparentes Dimethylperylimid-Pigment, das für den Einsatz in Metalliclacken geeignet ist. Die DE-B-2 504 481 beschreibt ein Verfahren zur Herstellung von transparenten Dimethylperylimid-Pigmenten, durch Kondensation von Perylen-3,4,9,10-tetracarbonsäuredianhydrid mit der mindestens 4-fach molaren Menge Monomethylamin. Das überschüssige Monomethylamin wird durch Filtration der Pigmentsuspension abgetrennt und kann aus der Mutterlauge mit Wasserdampf abdestilliert werden. Die Filtration der methylaminhaltigen Suspension bereitet erhebliche technische Probleme, da wegen der starken Geruchsbelästigung kein Monomethylamin in die Umwelt gelangen darf. Für die Aufarbeitung der methylaminhaltigen Mutterlauge muß eine zusätzliche Destillationsanlage bereitgestellt werden. Transparente Pigmente werden nur in großer Verdünnung erhalten.

Aus der EP-A-0208 266 ist die Herstellung eines deckenden Dimethylperylimid-Pigments bekannt. Bei diesem Verfahren wird feinteiliges, trockenes Dimethylperylimid-Rohpigment mit einer Teilchengröße von weniger als 0,04 µm in Gegenwart eines organischen Lösungsmittels in einer Mühle oder einem Dispergator einem rekristallisierenden Mahlprozeß unterworfen und das Dimethylperylimid-Pigment anschließend isoliert.

Die EP-A-0 088 392 beschreibt ein Verfahren zur Herstellung von transparenten Dimethylperylimid-Pigmenten durch Umsetzung von Persäure mit der mindestens 4-fach molaren Menge Monomethylamin. Das überschüssige Monomethylamin muß mit Natronlauge aus der Mutterlauge freigesetzt und destillativ abgetrennt werden. Hierbei fallen erhebliche Mengen Salze an, die das Abwasser belasten.

Die EP-A-0 318 022 beschreibt ein Verfahren zur Herstellung von deckenden Dimethylperylimid-Pigmenten durch Umsetzung von Persäure mit der mindestens 2-fach molaren Menge Monomethylamin. Das nach der Kondensation erhaltene Rohpigment wird auf einer Rührwerkskugelmühle gemahlen und anschließend mit Lösemitteln gefinisht. Transparente Pigmente können nach den dort beschriebenen Kondensations-, Perlmahl- und Finishbedingungen nicht erhalten werden.

Es bestand das Bedürfnis, transparente Dimethylperylimid-Pigmente in einfacher und ökologisch einwandfreier Weise herzustellen.

Es wurde gefunden, daß man transparente Dimethylperylimid-Pigmente mit günstigen coloristischen und rheologischen Eigenschaften in einfacher und ökologisch einwandfreier Weise aus Dimethylperylimid-Rohpigmenten durch Perlmahlung mit hoher Energiedichte herstellen kann, wenn spätestens bei der Perlmahlung Tenside und/oder Pigmentdispergatoren zugesetzt werden.

Gegenstand der Erfindung ist ein Verfahren zur Herstellung von N,N'-Dimethyl-perylen-3,4,9,10-tetracarbonsäurediimid unter Anwendung der Reaktion von Perylen-3,4,9,10-tetracarbonsäuredianhydrid mit Monomethylamin, dadurch gekennzeichnet, daß man pro Mol Perylen-3,4,9,10-tetracarbonsäuredianhydrid die mindestens 2-fach molare Menge Monomethylamin und eine mindestens 3-fache Gewichtsmenge Wasser, bezogen auf das Gewicht des Dianhydrids, einsetzt, die Reaktion bei einer Temperatur von 50 bis 200°C durchführt, nicht umgesetztes Monomethylamin abdestilliert, das erhaltene Rohpigment in einem flüssigen, wäßrigen oder wäßrig-organischen Medium auf einer Rührwerkskugelmühle, die mit einer Leistungsdichte von mehr als 1,5 kW pro Liter Mahlraum und einer Rührwerksumfangsgeschwindigkeit von mehr als 12 m/s betrieben wird, unter der Einwirkung von Mahlkörpem vom Durchmesser gleich oder kleiner als 0,9 mm naßvermahlt und das erhaltene Pigment isoliert; mit der Maßgabe, daß zu einem beliebigen Zeitpunkt des Verfahrens, spätestens jedoch während der Naßmahlung, ein oder mehrere Zusatzstoffe aus der Gruppe der Pigmentdispergatoren und Tenside zugegeben werden.

Das Perylen-3,4,9,10-tetracarbonsäuredianhydrid kann in trockener Form oder als Preßkuchen eingesetzt werden.

Vorzugsweise wird das erfindungsgemäße Verfahren unter Verwendung der 5- bis 12-fachen Gewichtsmenge Wasser, insbesondere mit der 6- bis 9-fachen Gewichtsmenge Wasser, bezogen auf das Gewicht des Dianhydrids, durchgeführt. Vorzugsweise wird eine 3- bis 10-fach molare Menge, insbesondere 3- bis 8-fach molare Menge Monomethylamin, pro Mol Dianhydrid eingesetzt. Das Monomethylamin wird vorzugsweise in wäßriger Lösung zugegeben, kann aber auch gasförmig eingeleitet werden.

Die Zugabe des Monomethylamins erfolgt zweckmäßigerweise bei einer Temperatur zwischen 0 und 80°C, bevorzugt zwischen 20 und 60°C. Die Umsetzung des Dianhydrids mit Monomethylamin erfolgt bei einer Temperatur zwischen 50 und 200°C, vorzugsweise zwischen 90 und 180°C, gegebenenfalls unter Druck. Nach Beendigung der Umsetzung wird überschüssiges Monomethylamin abdestilliert, vorteilhaft bei Normaldruck und bis zum Erreichen von etwa 100 °C am Destillationsübergang. Obwohl es vorteilhaft ist, alles überschüssige Monomethylamin vor dem Mahlprozeß zu entfernen, kann das Monomethylamin auch erst später entfernt werden, beispielsweise nach dem Mahlprozeß oder erst bei der Isolierung des Pigments.

Das abdestillierte Monomethylamin kann wieder für eine nächste Kondensationsreaktion eingesetzt werden.

Gegebenenfalls wird vor der Naßmahlung und/oder vor der Filtration mit etwas Säure neutralisiert, um Geruchsbelästigungen auszuschließen.

Das aus der Synthese erhaltene grobkristalline Perylenrohpigment wird zur Feinverteilung mit oder ohne Zwischenisolierung einer Naßmahlung zugeführt.

Für die erfindungsgemäße Herstellung dieser Pigmente ist eine hohe Mahlwirkung erforderlich, die durch den Einsatz spezieller Ausführungsformen einer Rührwerkskugelmühle erreicht wird. Für eine Mahlung der gesuchten Effizienz sind z.B. Rührwerkskugelmühlen geeignet, die zum diskontinuierlichen und kontinuierlichen Arbeitsablauf ausgelegt sind, mit zylinderförmigem oder hohlzylinderförmigem Mahlraum in horizontaler oder vertikaler Bauweise und die mit einer spezifischen Leistungsdichte von über 1,5 kW pro Liter Mahlraum betrieben werden können, wobei deren Rührwerksumfangsgeschwindigkeit über 12 m/s beträgt. Durch die bauliche Ausführung wird sichergestellt, daß die hohe Mahlenergie auf das Mahlgut übertragen wird. Dafür geeignete Mühlen sind z.B. in der DE-PS 3 716 587 beschrieben. Ist die Mahlintensität der Mühle zu gering, so werden die guten, erfindungsgemäßen Eigenschaften, insbesondere die hohe Transparenz und Farbstärke und hervorragende Coloristik der Pigmente nicht erreicht. Die pro Zeiteinheit vom Rührwerk abgegebene Energie wird als Zerkleinerungsarbeit und als Reibungsenergie in Form von Wärme auf das Mahlgut übertragen. Zur problemlosen Abführung dieser großen Wärmemenge muß durch konstruktive Vorkehrungen das Verhältnis von Mahlraum zu Mahlraumoberfläche (Kühlfläche) möglichst klein gehalten werden. Bei hohen Durchsätzen wird im Kreislauf gemahlen, und die Wärme wird überwiegend über das Mahlgut nach außen abgeführt. Als Mahlkörper dienen Kugeln aus Zirkonoxid, Zirkonmischoxid, Aluminiumoxid oder Quarz vom Durchmesser gleich oder kleiner als 0,9 mm; zweckmäßig werden solche mit einem Durchmesser von 0,2 bis 0,9 mm, vorzugsweise 0,3 bis 0,5 mm, verwendet.

Beim Einsatz von kontinuierlichen Rührwerkskugelmühlen zum Feinverteilen erfolgt die Abtrennung der Mahlkörper vom Mahlgut vorzugsweise durch Zentrifugalabscheidung, so daß die Trennvorrichtungen von den Mahlkörpem praktisch nicht berührt werden, wodurch man Verstopfungen derselben weitgehend verhindern kann. Die Rührwerkskugelmühlen werden hierbei mit hohem Mahlkörperfüllgrad betrieben. Bei den kontinuierlichen Rührwerkskugelmühlen wird der Mahlraum praktisch vollständig mit Mahlkörpern ausgefüllt.

Für die Mahlung werden verfahrensgemäß die bei der Synthese erhaltenen Rohpigmente eingesetzt, die nach dem Mahlvorgang als feinteilige Pigmente vorliegen.

Die Mahlung kann in einem wäßrigen, wäßrig-organischen oder organischen Medium durchgeführt werden. Vorzugsweise wird die Mahlung ohne Zusatz von Lösemitteln im wäßrigen alkalischen oder neutralen pH-Bereich durchgeführt. Die Pigmentkonzentration im Mahlgut ist abhängig von der Rheologie der Suspension. Sie sollte bei oder unter 30 % liegen, im allgemeinen 5 bis 25 % betragen, vorzugsweise zwischen 7,5 und 20 % sein.

Die Mahldauer ist abhängig von den Feinheitsanforderungen für das jeweilige Anwendungsgebiet. Deshalb liegt die Verweilzeit des Mahlguts in der Rührwerkskugelmühle je nach geforderter Feinheit im allgemeinen zwischen 5 und 60 Minuten. Sie beläuft sich normalerweise auf eine Dauer von 10 bis 45 Minuten, vorzugsweise 15 bis 30 Minuten.

Die Mahlung wird vorteilhaft bei Temperaturen im Bereich von 0 bis 100 °C, zweckmäßig bei einer Temperatur zwischen 10 und 60 °C, vorzugsweise bei 20 bis 50°C durchgeführt.

Das Mahlgut kann außer der flüssigen Phase, dem Rohpigment, der Tenside und/oder Pigmentdispergatoren noch andere Hilfsmittel, wie z.B. Füllstoffe, Stellmittel, Harze, Antischaummittel, Extender, Farbmittel, Konservierungsmittel und Trocknungsverzögerungsmittel enthalten.

Als flüssiges Mahlmedium werden Wasser, mit Wasser mischbare $C_1$-$C_8$-Alkanole wie z.B. Methanol, Ethanol, n-Propanol, Isopropanol, n-Butanol, tert.-Butanol, Isobutanol, Pentanol, Hexanol, Alkylhexanole, Ethylenglykol, Propylenglykol oder Glycerin; cyclische Alkanole wie z.B. Cyclohexanol; $C_1$-$C_5$-Dialkylketone wie z.B. Aceton, Diethylketon, Methylisobutylketon oder Methylethylketon; Ether und Glykolether wie z.B. Dimethoxyethan, Tetrahydrofuran, Methylglykol, Ethylglykol, Butylglykol, Ethyldiglykol, Methoxypropanol oder Methoxybutanol; aliphatische Säureamide wie

z.B. Dimethylacetamid oder Dimethylformamid; cyclische Carbonsäureamide wie z.B. N-Methylpyrrolidon, Valero- und Caprolactam, heterocyclische Basen wie z.B. Pyridin, Morpholin oder Picolin; sowie Dimethylsulfoxid oder Mischungen dieser Lösungsmittel mit Wasser verwendet. Besonders bevorzugt sind Wasser und Lösungen von $C_1$-$C_8$-Alkoholen in Wasser.

Die Zugabe der Tenside und/oder Pigmentdispergatoren und/oder der anderen Hilfsmittel erfolgt vorzugsweise vor der Kondensation oder vor der Perlmahlung. Aber auch während der Kondensation oder während der Mahlung können Tenside und/oder Pigmentdispergatoren und/oder andere Hilfsmittel auf einmal oder in mehreren Portionen zugegeben werden. Die Gesamtmenge der zugegebenen Tenside und/oder Pigmentdispergatoren beträgt zweckmäßigerweise 1 bis 25 Gew.-%, bevorzugt 5 bis 15 Gew.-%, bezogen auf das Rohpigment.

Als Tenside kommen anionaktive, kationaktive und nichtionogene Tenside oder Mischungen dieser Mittel in Betracht. Bevorzugt sind solche Tenside oder Mischungen von Tensiden, die bei der Destillation des Methylamins und bei der Naßmahlung nicht schäumen.

Als anionaktive oberflächenaktive Mittel kommen beispielsweise Fettsäuretauride, Fettsäure-N-methyltauride, Fettsäureisäthionate, Alkylbenzolsulfonate, Alkylnaphthalinsulfonate, Alkylphenolpolyglykolethersulfate und Fettalkoholpolyglykolethersulfate, Fettsäuren, z.B. Palmitin-, Stearin- und Ölsäure, Seifen, z.B. Alkalisalze von Fettsäuren, Naphthensäuren und Harzsäuren, z.B. Abietinsäure, alkalilösliche Harze, z.B. kolophoniummodifizierte Maleinatharze und Kondensationsprodukte auf Basis von Cyanurchlorid, Taurin, N,N'-Diethylaminopropylamin und p-Phenylendiamin in Betracht. Besonders bevorzugt sind Harzseifen, d.h. Alkalisalze von Harzsäuren.

Als kationaktive oberflächenaktive Mittel kommen beispielsweise quatemäre Ammoniumsalze, Fettaminoxäthylate, Fettaminopolyglykolether und Fettamine in Betracht.

Als nichtionogene oberflächenaktive Mittel kommen beispielsweise Aminoxide, Fettalkoholpolyglykolether, Fettsäurepolyglykolester und Alkylphenolpolyglykolether in Betracht.

Als Pigmentdispergatoren können Verbindungen der allgemeinen Formel (I),

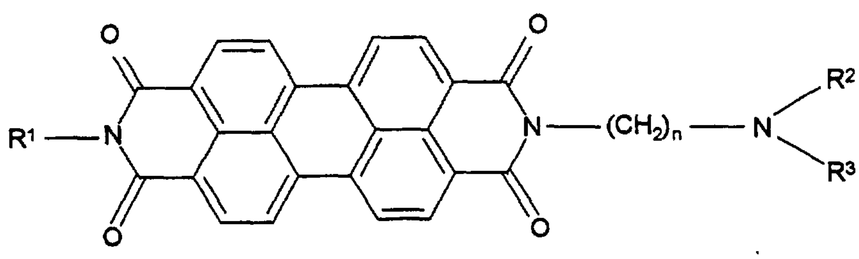 (I)

eingesetzt werden, in welcher

$R^1$ ein Wasserstoffatom, eine Hydroxy- oder Aminogruppe oder eine $C_1$-$C_{20}$-Alkylgruppe, die durch ein oder mehrere, z.B. 1, 2, 3 oder 4, Chlor- oder Bromatome, eine Phenylgruppe, ein oder mehrere, z.B. 1, 2, 3 oder 4, Cyan-, Hydroxy-, Carbamoyl-, $C_2$-$C_4$-Acyl- oder $C_1$-$C_4$-Alkoxygruppen, z.B. Methoxy oder Ethoxy, substituiert sein kann oder perfluoriert oder teilfluoriert ist; und

$R^2$ und $R^3$ unabhängig voneinander ein Wasserstoffatom, eine substituierte oder unsubstituierte, oder teil- oder perfluorierte Alkylgruppe mit 1 bis 20 Kohlenstoffatomen, oder eine substituierte oder unsubstituierte, oder teil- oder perfluorierte Alkenylgruppe mit 2 bis 20 Kohlenstoffatomen darstellen, wobei die Substituenten Hydroxy, Phenyl, Cyano, Chlor, Brom, $C_2$-$C_4$-Acyl oder $C_1$-$C_4$-Alkoxy sein und vorzugsweise 1 bis 4 an der Zahl sein können, oder $R^2$ und $R^3$ zusammen mit dem N-Atom einen gesättigten, ungesättigten oder aromatischen heterocyclischen Ring bilden, der gegebenenfalls ein weiteres Stickstoff-, Sauerstoff- oder Schwefelatom im Ring enthält; und

n eine Zahl von 1 bis 6 ist; und/oder es werden Verbindungen mit der allgemeinen Formel (II)

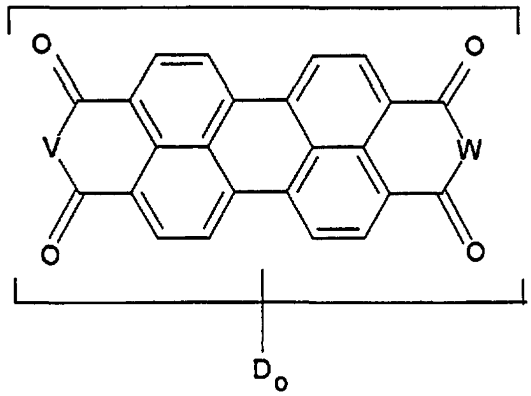

(II)

eingesetzt, in welcher
V einen bivalenten Rest -O-, >$NR^4$ oder >$NR^5$-$Y^-X^+$ und
W den bivalenten Rest >$NR^5$-$Y^-$ $X^+$ bedeutet,
D ein Chlor- oder Bromatom und sofern o > 1 ggf. eine Kombination davon darstellt, und o eine Zahl von 0 bis 4 ist;
$R^4$ für ein Wasserstoffatom oder eine $C_1$-$C_{18}$-Alkylgruppe, insbesondere $C_1$-$C_4$-Alkyl, oder für eine Phenylgruppe steht, die unsubstituiert oder durch Halogen wie Chlor oder Brom, $C_1$-$C_4$-Alkyl wie Methyl oder Ethyl, $C_1$-$C_4$-Alkoxy wie Methoxy oder Ethoxy, oder Phenylazo ein- oder mehrfach, z.B. 1, 2 oder 3-fach, substituiert sein kann,
$R^5$ für eine $C_1$-$C_{18}$-Alkylengruppe steht, die innerhalb der C-C-Kette durch ein Brückenglied aus der Reihe -O-, -$NR^6$-, -S-, Phenylen, -CO-, -$SO_2$- oder -$CR^7R^8$- oder eine chemisch sinnvolle Kombination davon ein- oder mehrfach, z.B. 1- bis 10-fach, unterbrochen sein kann und in dem die Bestandteile $R^6$, $R^7$ und $R^8$ unabhängig voneinander jeweils ein Wasserstoffatom oder eine $C_1$-$C_4$-Alkylgruppe sind, die unsubstituiert oder durch einen heterocyclischen Rest, vorzugsweise vom Imidazol- oder Piperazin-Typ, substituiert sein kann, insbesondere aber eine geradkettige oder verzweigte $C_1$-$C_6$-Alkylengruppe wie Ethylen oder Propylen ist;
oder $R^5$ für eine Phenylengruppe steht, die unsubstituiert oder durch $C_1$-$C_4$-Alkyl wie Methyl oder Ethyl, oder $C_1$-$C_4$-Alkoxy wie Methoxy oder Ethoxy ein- oder mehrfach, z.B. 1- bis 3-fach, substituiert sein kann,
$Y^-$ einen der anionischen Reste -$SO_3^-$oder -$COO^-$ bedeutet, und
$X^+$ die Bedeutung $H^+$ oder das Äquivalent

$$\frac{M^{m+}}{m}$$

eines Metallkations aus der 1. bis 5. Hauptgruppe oder aus der 1. oder 2. oder der 4. bis 8. Nebengruppe des Periodensystems der chemischen Elemente hat, wobei m eine der Zahlen 1, 2 oder 3 ist, wie z. B. $Li^{1+}$, $Na^{1+}$, $K^{1+}$, $Mg^{2+}$, $Ca^{2+}$, $Sr^{2+}$, $Ba^{2+}$, $Mn^{2+}$, $Cu^{2+}$, $Ni^{2+}$, $Co^{2+}$, $Zn^{2+}$, $Fe^{2+}$, $Al^{3+}$, $Cr^{3+}$ oder $Fe^{3+}$; oder ein Ammoniumion $N^+R^9R^{10}R^{11}R^{12}$ definiert, wobei die Substituenten $R^9$, $R^{10}$, $R^{11}$ und $R^{12}$ am quartären N-Atom unabhängig voneinander jeweils ein Wasserstoffatom oder eine Gruppe aus der Reihe $C_1$-$C_{30}$-Alkyl, $C_2$-$C_{30}$-Alkenyl, $C_5$-$C_{30}$-Cycloalkyl, unsubstituiertes oder durch $C_1$-$C_8$-alkyliertes Phenyl oder eine (Poly)alkylenoxygruppe

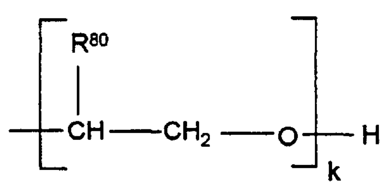

sind, in der $R^{80}$ für Wasserstoff oder $C_1$-$C_4$-Alkyl steht und k eine Zahl von 1 bis 30 ist;
und worin als $R^9$, $R^{10}$, $R^{11}$ und $R^{12}$ ausgewiesenes Alkyl, Alkenyl, Cycloalkyl, Phenyl oder Alkylphenyl durch Amino, Hydroxy und/oder Carboxy substituiert sein können;
oder wobei die Substituenten $R^9$ und $R^{10}$ zusammen mit dem quartären N-Atom ein fünf- bis siebengliedriges gesättigtes Ringsystem, das ggf. noch weitere Heteroatome wie ein O-, S- und/oder N-Atom enthält, bilden können,

z. B. vom Pyrrolidon-, Imidazolidin-, Hexamethylenimin-, Piperidin-, Piperazin- oder Morpholin-Typ;
oder wobei die Substituenten $R^9$, $R^{10}$ und $R^{11}$ zusammen mit dem quartären N-Atom ein fünf- bis siebengliedriges aromatisches Ringsystem, das ggf. noch weitere Heteroatome wie ein O-, S- und/oder N-Atom enthält und an dem ggf. zusätzliche Ringe ankondensiert sind, bilden können, z. B. vom Pyrrol-, Imidazol-, Pyridin-, Picolin-, Pyrazin-, Chinolin- oder Isochinolin-Typ;
und/oder es werden Verbindungen mit der allgemeinen Formel (III)

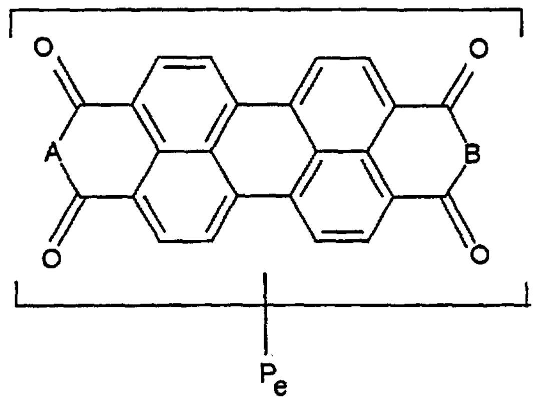

(III)

eingesetzt, in welcher
A einen kationischen bivalenten Rest der Formel

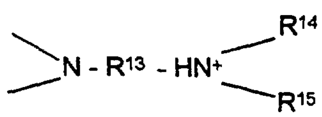

und
B einen anionischen bivalenten Rest der Formel

$\rangle N - R^{16} — SO_3^-$ oder $\rangle N - R^{16} - COO^-$

bedeutet,
e ein Wert von 0 bis 8, vorzugsweise von 1 bis 6 hat, und wenn e > 0 ist,
P ein Chlor- oder Bromatom und sofern e > 1 ist ggf. eine Kombination davon darstellt,
$R^{13}$ für eine $C_1$-$C_{12}$-Alkylengruppe, vorzugsweise eine $C_2$-$C_6$-Alkylengruppe, eine Aralkylengruppe oder eine Arylengruppe, vorzugsweise Phenylen oder Benzylen, steht,
$R^{14}$ und $R^{15}$ gleich oder verschieden sind und ein Wasserstoffatom, einen substituierten oder unsubstituierten $C_1$-$C_{20}$-Alkylrest, vorzugsweise einen $C_1$-$C_6$-Alkylrest, oder einen substituierten oder unsubstituierten $C_2$-$C_{20}$-Alkenylrest bedeuten, aber $R^{14}$ und $R^{15}$ nicht gleichzeitig Wasserstoff sind, oder $R^{14}$ und $R^{15}$ zusammen mit dem angrenzenden N-Atom ein heterocyclisches Ringsystem bilden, das 1, 2 oder 3 der Heteroatome O, S und/oder N im Ring enthält und an das ggf. zusätzliche Ringe ankondensiert sind, z.B. Chinolin oder Isochinolin, und
$R^{16}$ eine geradkettige oder verzweigte $C_1$-$C_{12}$-Alkylengruppe, vorzugsweise eine $C_1$-$C_6$-Alkylengruppe darstellt;
und/oder es werden Verbindungen mit der allgemeinen Formel (IV)

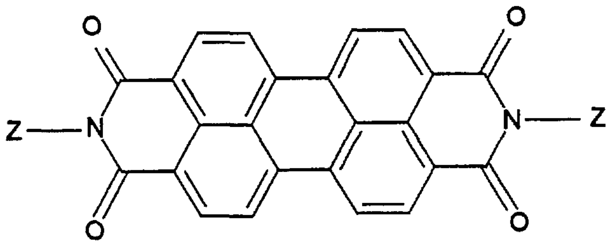

(IV)

eingesetzt, in welcher Z die Bedeutung $Z^1$, $Z^2$ oder $Z^3$ hat, mit der Maßgabe, daß beide Z nicht gleichzeitig $Z^3$ sind, wobei
$Z^1$ ein Rest der Formel (IVa) ist,

$$- [X- Y]_q-[X^1-Y^1]_r-[X^2-NH]_sH \qquad \text{(IVa)}$$

worin

X, $X^1$ und $X^2$ gleich oder verschieden sind und einen verzweigten oder unverzweigten $C_2$-$C_6$-Alkylenrest oder einen $C_5$-$C_7$-Cycloalkylenrest bedeuten, der durch 1 bis 4 $C_1$-$C_4$-Alkylreste, Hydroxyreste, Hydroxyalkylreste mit 1 bis 4 Kohlenstoffatomen und/oder durch 1 bis 2 weitere $C_5$-$C_7$-Cycloalkylreste substituiert sein kann;
Y und $Y^1$ gleich oder verschieden sind und eine NH-, -O-, N($C_1$-$C_6$-alkyl)-Gruppe,

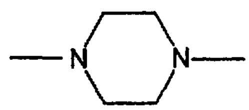

vorzugsweise $NCH_3$ oder bedeuten;
q eine Zahl von 1 bis 6, vorzugsweise 1, 2, 3 oder 4;
r und s unabhängig voneinander eine Zahl von 0 bis 6, vorzugsweise 0, 1 oder 2, wobei r und s nicht gleichzeitig Null sind;
$Z^2$ ein Rest der Formel (IVb) ist,

$$- [X - O]_{q1} - [X^1 - O]_q H \qquad \text{(IVb)}$$

worin

q1 eine Zahl von 0 bis 6; vorzugsweise 0, 1, 2, 3 oder 4; und
$Z^3$ Wasserstoff, Hydroxy, Amino oder $C_1$-$C_8$-Alkyl ist, wobei die Alkylgruppe durch 1 bis 4 Substituenten aus der Gruppe Cl, Br, CN, OH, $C_6H_5$, Carbamoyl, $C_1$-$C_4$-Acyl, $C_1$-$C_4$-Alkoxy und $NR^2R^3$ substituiert sein kann, oder perfluoriert oder teilfluoriert ist, wobei
$R^2$ und $R^3$ unabhängig voneinander ein Wasserstoffatom, eine substituierte oder unsubstituierte, oder teil- oder perfluorierte Alkylgruppe mit 1 bis 20 Kohlenstoffatomen, oder eine substituierte oder unsubstituierte, oder teil- oder perfluorierte Alkenylgruppe mit 2 bis 20 Kohlenstoffatomen darstellen, wobei die Substituenten Hydroxy, Phenyl, Cyano, Chlor, Brom, $C_2$-$C_4$-Acyl oder $C_1$-$C_4$-Alkoxy sein können, oder $R^2$ und $R^3$ zusammen mit dem N-Atom einen gesättigten, ungesättigten oder aromatischen heterocyclischen Ring bilden, der gegebenenfalls ein weiteres Stickstoff-, Sauerstoff- oder Schwefelatom im Ring enthält.

Vorzugsweise wird das Pigment direkt nach der Naßmahlung isoliert. Es ist aber möglich, eine Nachbehandlung (Finish) mit oder ohne organische Lösemittel durchzuführen, beispielsweise bei Temperaturen von 40 bis 160°C. Es ist auch möglich, die nach der Mahlung vorliegenden Pigmentsuspensionen einzudampfen oder sprühzutrocknen,

so daß eine Filtration entfallen kann.

Die Herstellung dieser Perylenpigmente nach dem erfindungsgemäßen Verfahren erfolgt ohne Abfallprodukte. Die wenigen Chemikalien können weiterverarbeitet oder wieder vollständig regeneriert werden.

Es war überraschend und nicht vorhersehbar, daß die Herstellung von transparenten Dimethylperylimid-Pigmenten in dieser einfachen und technisch eleganten Weise ohne ökologische Probleme möglich ist, da nach den bekannten Verfahren die Herstellung von transparenten Pigmenten nur in großer Verdünnung und mit erheblichen ökologischen Belastungen gelingt. Nach dem erfindungsgemäßen Verfahren hergestellte Perylenpigmente sind in ihren coloristischen und anwendungstechnischen Eigenschaften den nach bekannten Verfahren hergestellten Pigmenten deutlich überlegen.

Mit den nach dem erfindungsgemäßen Verfahren hergestellten Dimethylperylimid-Pigmenten können Automobillacke, insbesondere Metalliclacke mit hoher Pigmentkonzentration hergestellt werden. Es werden transparente und glänzende Lackierungen mit sehr guter Überlackierechtheit und Wetterechtheit erhalten.

Die Farbkonzentrate (mill bases) und die Lacke besitzen außerdem sehr gutes Fließverhalten bei hoher Pigmentkonzentration und eine hervorragende Flockungsstabilität.

Erfindungsgemäß hergestellte Perylenpigmente eignen sich besonders zum Pigmentieren von hochmolekularen natürlichen oder synthetischen organischen Materialien, wie beispielsweise Celluloseether und -ester, wie Ethylcellulose, Nitrocellulose, Celluloseacetat, Cellulosebutyrat, natürliche Harze oder Kunstharze, wie Polymerisationsharze oder Kondensationsharze, z.B. Aminoplaste, insbesondere Harnstoff- und Melamin-Formaldehydharze, Alkydharze, Acrylharze, Phenoplaste, Polycarbonate, Polyolefine, wie Polystyrol, Polyvinylchlorid, Polyethylen, Polypropylen, Polyacrylnitril, Polyacrylsäureester, Polyamide, Polyurethane oder Polyester, Gummi, Casein, Silikon und Silikonharze, einzeln oder in Mischungen.

Dabei spielt es keine Rolle, ob die erwähnten hochmolekularen organischen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemäß erhaltenen Pigmente als Blends oder in Form von Präparationen oder Dispersionen zu benutzen. Bezogen auf das zu pigmentierende, hochmolekulare organische Material setzt man die erfindungsgemäß hergestellten Pigmente in einer Menge von vorzugsweise 0,1 bis 10 % ein. Mit den nach dem erfindungsgemäßen Verfahren hergestellten Dimethylperylimid-Pigmenten können die technisch gängigen Einbrennlacke aus der Klasse der Alkyd-Melamin-Harzlacke, Acryl-Melamin-Harzlacke, Polyesterlacke, Highsolidacrylharzlacke, wäßrige Lacke auf Polyurethanbasis sowie Zweikomponentenlacke auf Basis polyisocyanatvernetzbarer Acrylharze und insbesondere Automobil-Metallic-Lacke pigmentiert werden.

Die erfindungsgemäß hergestellten Pigmente sind auch geeignet als Farbmittel in elektrophotographischen Tonern und Entwicklern, wie z. B. Ein- oder Zweikomponentenpulvertonern (auch Ein- oder Zweikomponenten-Entwickler genannt), Magnettoner, Flüssigtoner, Polymerisationstoner sowie Spezialtoner.

Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.

Des weiteren sind die erfindungsgemäß hergestellten Pigmente geeignet als Farbmittel in Pulver und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen. Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Außerdem sind die erfindungsgemäß hergestellten Pigmente als Farbmittel in Ink-Jet Tinten auf wäßriger und nichtwäßriger Basis sowie in solchen Tinten, die nach dem hot-melt-Verfahren arbeiten, geeignet. Darüber hinaus sind die erfindungsgemäß hergestellten Pigmente auch als Farbmittel für Farbfilter, sowohl für die subtraktive als auch für die additive Farberzeugung, geeignet.

Zur Beurteilung der Eigenschaften der nach der Erfindung hergestellten Pigmente auf dem Lacksektor wurden aus der Vielzahl der bekannten Lacke ein aromatenhaltiger Alkydmelaminharzlack (AM) auf Basis eines mittelöligen Alkydharzes und eines butanolveretherten Melaminharzes, ein Polyesterlack (PE) auf Basis von Celluloseacetobutyrat, ein High-Solid-Acrylharzeinbrennlack auf Basis einer nichtwäßrigen Dispersion (HS) sowie ein wäßriger Lack auf Polyurethanbasis (PUR) ausgewählt.

Die Bestimmung der Farbstärke und des Farbtons erfolgte nach DIN 55986.

Die Rheologie des Mahlguts nach der Dispergierung (mill base-Rheologie) wurde anhand der folgenden fünfstufigen Skala bewertet:

5 dünnflüssig
4 flüssig
3 dickflüssig
2 leicht gestockt
1 gestockt

Nach dem Verdünnen des Mahlguts auf die Pigmentendkonzentration wurde die Viskosität mit dem Viskospatel nach Rossmann, Typ 301 der Firma Erichsen beurteilt.
Glanzmessungen erfolgten an Folienaufgüssen unter einem Winkel von 20 ° nach DIN 67530 (ASTMD 523) mit dem "multigloss"-Glanzmeßgerät der Firma Byk-Mallinckrodt.
Die Bestimmung der Lösemittelechtheit erfolgte nach DIN 55976.
Die Bestimmung der Überlackierechtheit erfolgte nach DIN 53221.
In den nachstehenden Beispielen beziehen sich Teile jeweils auf Gewichtsteile und Prozente jeweils auf Gewichtsprozente der so beschriebenen Substanzen.

Beispiel 1

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und 2 Stunden bei Siedetemperatur gerührt. Danach wird eine wäßrige Lösung aus 7,5 Teilen Dimethyldistearylammoniumchlorid und 328 Teilen Wasser zugegeben und das Monomethylamin bis 100 °C am Übergang abdestilliert. Die Rohpigmentsuspension wird auf 20 °C abgekühlt.
Man erhält 1339,7 Teile Rohpigmentsuspension 12,4 %ig.
In eine Rührwerkskugelmühle (Hersteller: Draiswerke GmbH, Mannheim), die mit 410 Teilen Zirkonmischoxidperlen vom Durchmesser 0,3-0,4 mm als Mahlkörper gefüllt ist, werden 80,8 Teile der Rohpigmentsuspension 12,4 %ig und 19,2 Teile Wasser eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird anschließend abgesaugt, mit Wasser gewaschen und das erhaltene Pigment bei 80 °C getrocknet.
Man erhält 9,6 Teile Pigment. Im PE-Lack werden rote, transparente und farbstarke Lackierungen erhalten. Die Metalliclackierung ist farbstark und farbtief. Die Glanzmessung ergibt den Wert 47 und die Viskositätsmessung den Wert 3,5 s.

Vergleichsbeispiel 1a

(Wiederholung von Beispiel 1, aber Perlmahlung mit niedrigem Energieeintrag)

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und 2 Stunden bei Siedetemperatur gerührt. Danach wird eine wäßrige Lösung aus 7,5 Teilen Dimethyldistearylammoniumchlorid und 328 Teilen Wasser zugegeben und das Monomethylamin bis 100 °C am Übergang abdestilliert. Die Rohpigmentsuspension wird auf 20 °C abgekühlt.
Man erhält 1629 Teile Rohpigmentsuspension 10,0 %ig.
In eine Rührwerkskugelmühle mit Scheibenrührwerk (Hersteller: Draiswerke GmbH, Mannheim), die mit 1000 Teilen Quarzperlen vom Durchmesser 1 mm als Mahlkörper gefüllt ist, werden 350 Teile Rohpigmentsuspension 10,0 %ig eindosiert und mit einer Rührwerksumfangsgeschwindigkeit von 10,2 m/s sowie einer spezifischen Leistungsdichte von 0,45 kW pro Liter Mahlraum 1 Stunde lang bei 25 °C gemahlen. Anschließend wird die Mahlgutsuspension von den Mahlkörpem abgesiebt, die Mahlkörper mit Wasser abgespült und die Mahlgutsuspensionen vereinigt, abgesaugt, mit Wasser gewaschen und bei 80°C getrocknet.
Man erhält 33,9 Teile Pigment. Im PE-Lack werden deckende Lackierungen erhalten. Das Pigment ist daher nicht für Metalliclacke geeignet.

Vergleichsbeispiel 1b

(Wiederholung von Beispiel 1, aber Perlmahlung mit niedrigem Energieeintrag und 0,3 - 0,4 mm-Mahlkörpern)

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und 2 Stunden bei Siedetemperatur gerührt. Danach wird eine wäßrige Lösung aus 7,5 Teilen Dimethyldistearylammoniumchlorid und 328 Teilen Wasser zugegeben und das Monomethylamin bis 100 °C am Übergang abdestilliert. Die Rohpigmentsuspension wird auf 20 °C abgekühlt.
Man erhält 1629 Teile Rohpigmentsuspension 10,0 %ig.
In eine Rührwerkskugelmühle mit Scheibenrührwerk (Hersteller: Draiswerke GmbH, Mannheim), die mit 1500 Teilen Zirkonmischoxidperlen vom Durchmesser 0,3-0,4 mm als Mahlkörper gefüllt ist, werden 350 Teile Rohpigmentsuspension 10,0 %ig eindosiert und mit einer Rührwerksumfangsgeschwindigkeit von 10,2 m/s sowie einer spezifischen Leistungsdichte von 0,45 kW pro Liter Mahlraum 1 Stunde lang bei 25 °C gemahlen. Anschließend wird die Mahlgutsuspension von den Mahlkörpern abgesiebt, die Mahlkörper mit Wasser abgespült und die Mahlgutsuspensionen vereinigt, abgesaugt, mit Wasser gewaschen und bei 80 °C getrocknet.
Man erhält 33,5 Teile Pigment. Im PE-Lack werden deckende Lackierungen erhalten. Das Pigment ist daher nicht für Metalliclacke geeignet.

Vergleichsbeispiel 1c

(Wiederholung von Beispiel 1 unter Verzicht der Perlmahlung)

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und zwei Stunden bei Siedetemperatur gerührt. Danach wird eine wäßrige Lösung aus 7,5 Teilen Dimethyldistearylammoniumchlorid und 328 Teilen Wasser zugegeben und das Monomethylamin bis 100 °C am Übergang abdestilliert. Die Pigmentsuspension wird auf 20 °C abgekühlt, abgesaugt, mit Wasser gewaschen und das erhaltene Perylendiimid bei 80 °C getrocknet.
Man erhält 165,9 Teile N,N-Dimethyl-perylen-3,4,9-10-tetracarbonsäurediimid. Im PE-Lack werden deckende und farbschwache Lackierungen erhalten.
Die Metalliclackierung ist farbschwach und matt. Die Glanzmessung ergibt den Wert 5 und die Viskositätsmessung den Wert 1,9 s.

Beispiel 2

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und 2 Stunden bei Siedetemperatur gerührt. Danach wird eine wäßrige Lösung aus 7,5 Teilen Dimethyldistearylammoniumchlorid und 328 Teilen Wasser zugegeben und das Monomethylamin bis 100 °C am Übergang abdestilliert. Die Rohpigmentsuspension wird auf 20 °C abgekühlt.
Man erhält 1339,7 Teile Rohpigmentsuspension 12,4 %ig.
In eine Rührwerkskugelmühle (Hersteller: Draiswerke GmbH, Mannheim), die mit 419 Teilen Zirkonmischoxidperlen vom Durchmesser 0,3-0,4 mm als Mahlkörper gefüllt ist, werden 60,6 Teile der Rohpigmentsuspension 12,4 %ig und 39,4 Teile Wasser eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird anschließend abgesaugt, mit Wasser gewaschen und das erhaltene Pigment bei 80°C getrocknet.
Man erhält 7,2 Teile Pigment. Im PE-Lack werden transparente und farbstarke Lackierungen erhalten. Die Metalliclackierung ist farbstark und rein. Die Glanzmessung ergibt den Wert 33 und die Viskositätsmessung den Wert 4,0 s.

Beispiel 3

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und 2 Stunden bei Siedetemperatur

gerührt. Danach werden 7,5 Teile Dimethyldistearylammoniumchlorid zugegeben und das Monomethylamin bis 100°C am Übergang abdestilliert. Die Rohpigmentsuspension wird auf 20°C abgekühlt. Man erhält 1676 Teile Rohpigmentsuspension 10,4 %ig.
In eine Rührwerkskugelmühle (Hersteller: Draiswerke GmbH, Mannheim), die mit 360 Teilen Zirkonmischoxidperlen vom Durchmesser 0,3-0,4 mm als Mahlkörper gefüllt ist, werden 100 Teile der Rohpigmentsuspension 10,4 %ig eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird durch Zugabe von Essigsäure auf pH 5-6 gestellt, anschließend abgesaugt, mit Wasser gewaschen und das erhaltene Pigment bei 80 °C getrocknet.
Man erhält 8,4 Teile Pigment. Im PE-Lack werden transparente und farbstarke Lackierungen erhalten. Die Metalliclackierung ist farbstark und rein. Die Glanzmessung ergibt den Wert 33 und die Viskositätsmessung den Wert 2,9 s.

Beispiel 4

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und 2 Stunden bei Siedetemperatur gerührt. Danach werden 7,5 Teile Dimethyldistearylammoniumchlorid zugegeben und das Monomethylamin bis 100 °C am Übergang abdestilliert. Die Rohpigmentsuspension wird auf 20 °C abgekühlt. Man erhält 1676 Teile Rohpigmentsuspension 10,4 %ig.
In eine Rührwerkskugelmühle (Hersteller: Draiswerke GmbH, Mannheim), die mit 360 Teilen Zirkonmischoxidperlen vom Durchmesser 0,3-0,4 mm als Mahlkörper gefüllt ist, werden 100 Teile der Rohpigmentsuspension 10,4 %ig eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird durch Zugabe von Essigsäure auf pH 8 gestellt, anschließend abgesaugt, mit Wasser gewaschen und das erhaltene Pigment bei 80 °C getrocknet.
Man erhält 8,7 Teile Pigment. Im PE-Lack werden transparente und farbstarke Lackierungen erhalten. Die Metalliclackierung ist farbstark. Die Glanzmessung ergibt den Wert 26 und die Viskositätsmessung den Wert 2,7 s.

Beispiel 5

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und zwei Stunden bei Siedetemperatur gerührt. Danach werden 7,5 Teile Dimethyldistearylammoniumchlorid zugegeben und das Monomethylamin bis 100 °C am Übergang abdestilliert. Die Rohpigmentsuspension wird auf 20 °C abgekühlt. Man erhält 1676 Teile Rohpigmentsuspension 10,4 %ig.
In eine Rührwerkskugelmühle (Hersteller: Draiswerke GmbH, Mannheim), die mit 342 Teilen Zirkonmischoxidperlen vom Durchmesser 0,3-0,4 mm als Mahlkörper gefüllt ist, werden 100 Teile der Rohpigmentsuspension 10,4 %ig eindosiert und 30 Minuten lang mit einer Umfangsgeschwindigkeit von 17,7 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird durch Zugabe von Essigsäure auf pH 5-6 gestellt, anschließend abgesaugt, mit Wasser gewaschen und das erhaltene Pigment bei 80 °C getrocknet.
Man erhält 10 Teile Pigment. Im PE-Lack werden transparente und farbstarke Lackierungen erhalten. Die Metalliclackierung ist farbstark und rein. Die Glanzmessung ergibt den Wert 50.

Beispiel 6

In einem Rührgefäß werden 1350 Teile Wasser vorgelegt und unter Rühren bei 25 °C nacheinander 150 Teile Perylen-3,4,9,10-tetracarbonsäuredianhydrid, 249,5 Teile einer 40 %igen wäßrigen Monomethylaminlösung und 15 Teile Pigmentdispergator mit der allgemeinen Formel (III) zugegeben. Im bivalenten Rest A dieser Formel bedeutet $R^{13}$ eine Propylengruppe, $R^{14}$ und $R^{15}$ je eine Ethylgruppe und in dem bivalenten Rest B dieser Formel bedeutet $R^{16}$ eine Methylengruppe und e ist die Zahl 0. Der Pigmentdispergator liegt als inneres Salz vor. Es wird zum Sieden erhitzt und 2 Stunden bei Siedetemperatur gerührt. Danach werden 28,2 Teile einer 29 %igen wäßrigen Lösung von Hexadecyltrimethylammoniumchlorid zugegeben und das Monomethylamin bis 100 °C am Übergang abdestilliert. Die Rohpigmentsuspension wird auf 20 °C abgekühlt.
Man erhält 1599 Teile Rohpigmentsuspension 10,4 %ig.
In eine Rührwerkskugelmühle (Hersteller: Draiswerke GmbH, Mannheim), die mit 360 Teilen Zirkonmischoxidperlen

vom Durchmesser 0,3-0,4 mm als Mahlkörper gefüllt ist, werden 86,5 Teile der Rohpigmentsuspension 10,4 %ig eindosiert und 30 Minuten lang mit einer Umfangsgeschwindigkeit von 15,8 m/s und einer Leistungsdichte von 3,5 kW/l Mahlraum bei 20 ° gemahlen. Danach werden die Mahlkörper durch Sieben vom Mahlgut abgetrennt. Die Mahlgutsuspension wird anschließend abgesaugt, mit Wasser gewaschen und die erhaltene Pigmentzubereitung bei 80 °C getrocknet.
Man erhält 7,7 Teile Pigmentzubereitung. Im HS-Lack werden marronfarbene, transparente und farbstarke Lackierungen erhalten. Die Glanzmessung ergibt den Wert 75. Die Pigmentzubereitung ist nicht geflockt.

Beispiel 7

In einem Rührgefäß werden 1350 Teile Wasser vorgelegt und unter Rühren bei 60 °C nacheinander 150 Teile Perylen-3,4,9,10-tetracarbonsäuredianhydrid, 15 Teile einer 50 %igen wäßrigen Harzseife und 89 Teile einer 40 %igen wäßrigen Monomethylaminlösung zugegeben. Es wird zum Sieden erhitzt und 2 Stunden bei Siedetemperatur gerührt. Danach wird eine wäßrige Lösung aus 7,5 Teilen Dimethyldistearylammoniumchlorid und 328 Teilen Wasser zugegeben und das Monomethylamin bis 100 °C am Übergang abdestilliert. Anschließend wird auf 125 °C erhitzt und 3 Stunden bei 125 °C gerührt. Nach beendeter Reaktion wird die Rohpigmentsuspension auf 20 °C abgekühlt.
Man erhält 1537 Teile Rohpigmentsuspension 9,0 %ig.
In eine Rührwerkskugelmühle (Hersteller: Draiswerke GmbH, Mannheim), die mit 360 Teilen Zirkonmischoxidperlen vom Durchmesser 0,3-0,4 mm als Mahlkörper gefüllt ist, werden 100 Teile der Rohpigmentsuspension 9,0 %ig eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird abgesaugt, mit Wasser gewaschen und das erhaltene Pigment bei 80 °C getrocknet.
Man erhält 7,5 Teile Pigment. Im PE-Lack werden transparente und farbstarke Lackierungen erhalten. Die Metalliclakkierung ist farbstark und tief. Die Glanzmessung ergibt den Wert 63 und die Viskositätsmessung den Wert 3,4 s. Die Rheologie wird mit 3 bewertet.

Beispiel 8

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile Pigmentdispergator der allgemeinen Formel (II) und 249,5 Teile einer 40%igen wäßrigen Monomethylaminlösung zugegeben. In den bivalenten Resten V und W dieser Formel bedeutet $R^5$ je eine Ethylengruppe, $Y^-$ den Rest $-SO_3^-$, $X^+$ ein Proton und o ist die Zahl 0. Es wird zum Sieden erhitzt und 2 Stunden bei Siedetemperatur gerührt. Danach wird das Monomethylamin bis 100 °C am Übergang abdestilliert. Danach werden 52,6 Teile einer 29 %igen wäßrigen Lösung von Hexadecyltrimethylammoniumchlorid zugegeben. Die Rohpigmentsuspension wird auf 20 °C abgekühlt.
Man erhält 1664 Teile Rohpigmentsuspension 11,1 %ig.
In eine Rührwerkskugelmühle (Hersteller: Draiswerke GmbH, Mannheim), die mit 342 Teilen Zirkonmischoxidperlen vom Durchmesser 0,3-0,4 mm als Mahlkörper gefüllt ist, werden 100 Teile der Rohpigmentsuspension 11,1 %ig eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird abgesaugt, mit Wasser gewaschen und die erhaltene Pigmentzubereitung bei 80 °C getrocknet.
Man erhält 9,3 Teile Pigmentzubereitung. Im HS-Lack werden transparente und farbstarke Lackierungen erhalten. Die Glanzmessung ergibt den Wert 77.

Beispiel 9

In einem Rührgefäß werden 1350 Teile Wasser vorgelegt und unter Rühren bei 50 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und zwei Stunden bei Siedetemperatur gerührt. Danach wird eine wäßrige Lösung aus 7,5 Teilen Dimethyldistearylammoniumchlorid und 328 Teilen Wasser zugegeben und das Monomethylamin bis 100 °C am Übergang abdestilliert. Die Pigmentsuspension wird auf 20 °C abgekühlt.
Man erhält 1482 Teile Rohpigmentsuspension 10,8 %ig.
In eine Rührwerkskugelmühle (Hersteller: Draiswerke GmbH, Mannheim), die mit 364 Teile Zirkonmischoxidperlen vom Durchmesser 0,3-0,4 mm als Mahlkörper gefüllt ist, werden 92,6 Teile der Rohpigmentsuspension 10,8 %ig und 7,4 Teile Wasser eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird anschließend abgesaugt, mit Wasser gewaschen und das erhaltene Pigment bei

80 °C getrocknet.
Man erhält 8,9 Teile Pigment. Im PE-Lack werden transparente und farbstarke Lackierungen erhalten. Die Metalliclakkierung ist farbstark und farbtief. Die Glanzmessung ergibt den Wert 70 und die Viskositätsmessung 3,4 s. Die Lösemittelechtheit ist einwandfrei.

Beispiel 10

(Wiederholung von Beispiel 1, aber Zugabe von Harzseife und Dimethyldistearylammoniumchlorid erst bei der Mahlung)

In einem Rührgefäß werden 1350 Teile Wasser vorgelegt und unter Rühren bei 25°C nacheinander 150 Teile Perylen-3,4,9,10-tetracarbonsäuredianhydrid und 249,5 Teile einer 40 %igen wäßrigen Monomethylaminlösung zugegeben. Es wird zum Sieden erhitzt und 2 Stunden bei Siedetemperatur gerührt. Danach wird das Monomethylamin bis 100 °C am Übergang abdestilliert. Die Rohpigmentsuspension wird auf 20 °C abgekühlt.
Man erhält 1662 Teile Rohpigmentsuspension 9,6 %ig.
In eine Rührwerkskugelmühle (Hersteller: Draiswerke GmbH, Mannheim), die mit 416 Teile Zirkonmischoxidperlen vom Durchmesser 0,3-0,4 mm als Mahlkörper gefüllt ist, werden 98,6 Teile der Rohpigmentsuspension 9,6 %ig, 0,9 Teile einer 50 %igen wäßrigen Harzseife und 0,45 Teile Dimethyldistearylammoniumchlorid eindosiert und 30 Minuten lang mit einer Umfangsgeschwindigkeit von 15,8 m/s und einer Leistungsdichte von 3,5 kW/l Mahlraum bei 20°C gemahlen. Danach werden die Mahlkörper durch Sieben vom Mahlgut abgetrennt. Die Mahlgutsuspension wird anschließend abgesaugt, mit Wasser gewaschen und das erhaltene Pigment bei 80 °C getrocknet.
Man erhält 9,4 Teile Pigment. Im PE-Lack werden transparente und farbstarke Lackierungen erhalten. Die Metalliclakkierung ist transparent und farbstark. Die Glanzmessung ergibt den Wert 59 und die Viskositätsmessung den Wert 2,3 s. Die Rheologie wird mit 4 bewertet.

Vergleichsbeispiel 10a

(Wiederholung von Beispiel 10, aber Kondensation und Perlmahlung ohne Harzseife und Dimethyldistearylammoniumchlorid)

In einem Rührgefäß werden 1350 Teile Wasser vorgelegt und unter Rühren bei 25 °C nacheinander 150 Teile Perylen-3,4,9,10-tetracarbonsäuredianhydrid und 249,5 Teile einer 40 %igen wäßrigen Monomethylaminlösung zugegeben. Es wird zum Sieden erhitzt und 2 Stunden bei Siedetemperatur gerührt. Danach wird das Monomethylamin bis 100 °C am Übergang abdestilliert. Die Rohpigmentsuspension wird auf 20 °C abgekühlt.
Man erhält 1662 Teile Rohpigmentsuspension 9,6 %ig.
In eine Rührwerkskugelmühle (Hersteller: Draiswerke GmbH, Mannheim), die mit 420 Teile Zirkonmischoxidperlen vom Durchmesser 0,3-0,4 mm als Mahlkörper gefüllt ist, werden 98,6 Teile der Rohpigmentsuspension 9,6 %ig eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird anschließend abgesaugt, mit Wasser gewaschen und das erhaltene Pigment bei 80 °C getrocknet.
Man erhält 9,4 Teile Pigment. Im PE-Lack werden deckende und farbschwache Lackierungen erhalten. Die Metalliclackierung ist farbschwach und trüb. Die Glanzmessung ergibt den Wert 5 und die Viskositätsmessung den Wert 2,4 s.

Vergleichsbeispiel 10b

(Wiederholung von Beispiel 10, aber Harzseife und Dimethyldistearylammoniumchlorid erst nach der Perlmahlung zugegeben).

In einem Rührgefäß werden 1350 Teile Wasser vorgelegt und unter Rühren bei 25 °C nacheinander 150 Teile Perylen-3,4,9,10-tetracarbonsäuredianhydrid und 249,5 Teile einer 40 %igen wäßrigen Monomethylaminlösung zugegeben. Es wird zum Sieden erhitzt und 2 Stunden bei Siedetemperatur gerührt. Danach wird das Monomethylamin bis 100 °C am Übergang abdestilliert. Die Rohpigmentsuspension wird auf 20 °C abgekühlt.
Man erhält 1662 Teile Rohpigmentsuspension 9,6 %ig.
In eine Rührwerkskugelmühle (Hersteller: Draiswerke GmbH, Mannheim), die mit 420 Teilen Zirkonmischoxidperlen vom Durchmesser 0,3-0,4 mm als Mahlkörper gefüllt ist, werden 98,6 Teile der Rohpigmentsuspension 9,6 %ig eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. In die Mahlgutsuspension werden bei 25 °C nacheinander 0,9 Teile einer 50 %igen wäßrigen Harzseife und 0,45 Teile Dimethyldistearylammoniumchlorid zugegeben und 1 Stunde bei 25 °C gerührt. Anschließend wird das Pigment abgesaugt, mit Wasser gewaschen und das erhaltene Pigment bei 80 °C getrocknet.
Man erhält 9,2 Teile Pigment. Im PE-Lack werden deckende Lackierungen erhalten.

Beispiel 11

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und 2 Stunden bei Siedetemperatur gerührt. Danach wird eine wäßrige Lösung aus 7,5 Teilen Dimethyldistearylammoniumchlorid und 328 Teilen Wasser zugegeben und das Monomethylamin bis 100 °C am Übergang abdestilliert. Die Rohpigmentsuspension wird auf 20 °C abgekühlt.
Man erhält 1339,7 Teile Rohpigmentsuspension 12,4 %ig.
In eine Rührwerkskugelmühle (Hersteller: Draiswerke GmbH, Mannheim), die mit 357 Teile Zirkonmischoxidperlen vom Durchmesser 0,3-0,4 mm als Mahlkörper gefüllt ist, werden 80,8 Teile der Rohpigmentsuspension 12,4 %ig und 19,2 Teile Wasser eindosiert und 10 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird anschließend abgesaugt, mit Wasser gewaschen und das erhaltene Pigment bei 80 °C getrocknet.
Man erhält 9,7 Teile Pigment. Im PE-Lack werden transparente und farbstarke Lackierungen erhalten. Die Metalliclakkierung ist farbstark und farbtief. Die Glanzmessung ergibt den Wert 86 und die Viskositätsmessung den Wert 3,5 s.

Beispiel 12

In einem Rührgefäß werden 1350 Teile Wasser vorgelegt und unter Rühren bei 25 °C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben. Es wird zum Sieden erhitzt und zwei Stunden bei Siedetemperatur gerührt. Danach wird eine wäßrige Lösung aus 7,5 Teilen Dimethyldistearylammoniumchlorid und 328 Teilen Wasser zugegeben und das Monomethylamin bis 100 °C am Übergang abdestilliert. Die Rohpigmentsuspension wird auf 20 °C abgekühlt.
Man erhält 1339,7 Teile Rohpigmentsuspension 12,4 %ig.
In eine Rührwerkskugelmühle (Hersteller: Draiswerke GmbH, Mannheim), die mit 368 Teilen Zirkonmischoxidperlen vom Durchmesser 0,3-0,4 mm als Mahlkörper gefüllt ist, werden 80,8 Teile der Rohpigmentsuspension 12,4 %ig, 14,7 Teile Wasser und 4,5 Teile Ethanol eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird anschließend abgesaugt, mit Wasser gewaschen und das erhaltene Pigment bei 80 °C getrocknet.
Man erhält 10,0 Teile Pigment. Im AM-Lack werden transparente und farbstarke Lackierungen erhalten. Die Glanzmessung ergibt den Wert 84. Die Überlackierechtheit ist einwandfrei.

Beispiel 13

In einem Rührgefäß werden 1350 Teile Wasser vorgelegt und unter Rühren bei 25 °C nacheinander 150 Teile Perylen-3,4,9,10-tetracarbonsäuredianhydrid, 72,8 Teile eines Kondensationsproduktes auf Basis von Cyanurchlorid, Taurin, N,N'-Diethylaminopropylamin und p-Phenylendiamin 22,4 %ig und 249,5 Teile einer 40 %igen wäßrigen Monomethylaminlösung zugegeben und 1 Stunde bei 25 °C gerührt. Danach wird zum Sieden erhitzt und 2 Stunden bei Siedetemperatur gerührt. Dann wird das Monomethylamin bis 100 °C am Übergang abdestilliert. Die Rohpigmentsuspension wird auf 20 °C abgekühlt.
Man erhält 1392 Teile Rohpigmentsuspension 11,7 %ig.
In eine Rührwerkskugelmühle (Hersteller: Draiswerke GmbH, Mannheim), die mit 402 Teile Zirkonmischoxidperlen vom Durchmesser 0,3-0,4 mm als Mahlkörper gefüllt ist, werden 85,2 Teile der Rohpigmentsuspension 11,7 %ig und 14,8 Teile Wasser eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird anschließend im Vakuum zur Trockene eingedampft.
Man erhält 10,4 Teile Pigment.
7,6 Teile Pigment werden mit 0,4 Teilen Pigmentdispergator der allgemeinen Formel (II) mechanisch gemischt. Im bivalenten Rest V dieser Formel bedeutet $R^4$ eine Methylgruppe und in dem bivalenten Rest W bedeutet $R^5$ eine Methylengruppe, $Y^-$ den Rest $-SO_3^-$, $X^+$ ein Wasserstoffion und o die Zahl 0.

Man erhält eine Pigmentzubereitung. Im PUR-Lack werden transparente und farbstarke Lackierungen erhalten. Die Metalliclackierung ist farbstark.

Beispiel 14

In einem Rührgefäß werden 750 Teile Wasser vorgelegt und unter Rühren bei 25°C nacheinander 150 Teile Perylen-3,4,9,10-tetracarbonsäuredianhydrid, 15 Teile einer 50 %igen wäßrigen Harzseife und 249,5 Teile einer 40 %igen wäßrigen Monomethylaminlösung zugegeben und 1 Stunde bei 25 °C gerührt. Danach wird eine wäßrige Lösung aus 7,5 Teilen Dimethyldistearylammoniumchlorid und 328 Teilen Wasser zugegeben. Es wird zum Sieden erhitzt, 2 Stunden bei Siedetemperatur gerührt und dann das Monomethylamin bis 100 °C am Übergang abdestilliert. Die Rohpigmentsuspension wird auf 20°C abgekühlt.
Man erhält 1416 Teile Rohpigmentsuspension 11,3 %ig.
In eine Rührwerkskugelmühle (Hersteller: Draiswerke GmbH, Mannheim), die mit 366 Teile Zirkonmischoxidperlen vom Durchmesser 0,3-0,4 mm als Mahlkörper gefüllt ist, werden 88,2 Teile der Rohpigmentsuspension 11,3 %ig und 11,8 Teile Wasser eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird anschließend abgesaugt, mit Wasser gewaschen und das erhaltene Pigment bei 80 °C getrocknet.
Man erhält 8,5 Teile Pigment. Im PE-Lack werden rote, transparente und farbstarke Lackierungen erhalten. Die Metalliclackierung ist farbstark, farbtief und rein. Die Glanzmessung ergibt den Wert 68 und die Viskositätsmessung den Wert 3,8 s.

Beispiel 15

In einem Rührgefäß werden 879 Teile Wasser vorgelegt und unter Rühren bei 25 °C nacheinander 100 Teile Perylen-3,4,9,10-tetracarbonsäuredianhydrid, 26,1 Teile Pigmentdispergator der allgemeinen Formel (IV) als 19,1 %iger Preßkuchen und 166,3 Teile einer wäßrigen 40 %igen Monomethylaminlösung zugegeben. In dieser Formel (IV) bedeutet Z eine Hydroxyethylengruppe und $Z^1$ den Rest der Formel IVa, in welcher X und $X^2$ jeweils einen Propylenrest und Y eine NH-Gruppe bedeutet, q und s die Zahl 1 und r die Zahl 0 darstellt. Es wird zum Sieden erhitzt, 2 Stunden bei Siedetemperatur gerührt und das Monomethylamin bis 100 °C am Übergang abdestilliert. Die Rohpigmentsuspension wird auf 20 °C abgekühlt.
Man erhält 985,9 Teile Rohpigmentsuspension 11,2 %ig.
In eine Rührwerkskugelmühle (Hersteller: Draiswerke GmbH, Mannheim), die mit 360 Teile Zirkonmischoxidperlen vom Durchmesser 0,3-0,4 mm als Mahlkörper gefüllt ist, werden 80,8 Teile der Rohpigmentsuspension 12,4 %ig und 19,2 Teile Wasser eindosiert und 30 Minuten lang mit einer Umfangsgeschwindigkeit von 15,8 m/s und einer Leistungsdichte von 3,5 kW/l Mahlraum bei 20 °C gemahlen. Danach werden die Mahlkörper durch Sieben vom Mahlgut abgetrennt. Die Mahlgutsuspension wird anschließend abgesaugt, mit Wasser gewaschen und das erhaltene Pigment bei 80 °C getrocknet.
Man erhält 8,9 Teile Pigment. Im HS-Lack werden transparente und farbstarke Lackierungen erhalten. Die Glanzmessung ergibt den Wert 73 und die Viskositätsmessung den Wert 3,5 s. Die Rheologie wird mit 3 bewertet.

## Patentansprüche

1. Verfahren zur Herstellung von N,N'-Dimethyl-perylen-3,4,9,10-tetracarbonsäurediimid unter Anwendung der Reaktion von Perylen-3,4,9,10-tetracarbonsäuredianhydrid mit Monomethylamin, **dadurch gekennzeichnet, daß** man pro Mol Perylen-3,4,9,10- tetracarbonsäuredianhydrid die mindestens 2-fach molare Menge Monomethylamin und eine mindestens 3-fache Gewichtsmenge Wasser, bezogen auf das Gewicht des Dianhydrids, einsetzt, die Reaktion bei einer Temperatur von 50 bis 200 °C durchführt, nicht umgesetztes Monomethylamin abdestilliert, das erhaltene Rohpigment in einem flüssigen, wäßrigen oder wäßrig-organischen Medium auf einer Rührwerkskugelmühle, die mit einer Leistungsdichte von mehr als 1,5 kW pro Liter Mahlraum und einer Rührwerksumfangsgeschwindigkeit von mehr als 12 m/s betrieben wird, unter der Einwirkung von Mahlkörpern vom Durchmesser gleich oder kleiner als 0,9 mm naßvermahlt und das erhaltene Pigment isoliert; mit der Maßgabe, daß zu einem beliebigen Zeitpunkt des Verfahrens, spätestens jedoch während der Naßmahlung, ein oder mehrere Zusatzstoffe aus der Gruppe der Pigmentdispergatoren und Tenside zugegeben werden.

2. Verfahren nach Anspruch 1, **dadurch gekennzeichnet, daß** man pro Mol Perylen-3,4,9,10-tetracarbonsäuredianhydrid die 3- bis 10-fach molare Menge, vorzugsweise 3- bis 8-fach molare Menge, Monomethylamin einsetzt.

**3.** Verfahren nach Anspruch 1 oder 2, **dadurch gekennzeichnet, daß** man das Monomethylamin bei 0 bis 80 °C, vorzugsweise bei 20 bis 60 °C, zusetzt.

**4.** Verfahren nach mindestens einem der Ansprüche 1 bis 3, **dadurch gekennzeichnet, daß** man eine 5- bis 12-fache, vorzugsweise 6- bis 9-fache, Gewichtsmenge Wasser, bezogen auf das Gewicht des Dianhydrids, einsetzt.

**5.** Verfahren nach mindestens einem der Ansprüche 1 bis 4, **dadurch gekennzeichnet, daß** man das Rohpigment in wäßriger Suspension ohne Zwischenisolierung der Naßmahlung zuführt.

**6.** Verfahren gemäß einem oder mehreren der Ansprüche 1 bis 5, **dadurch gekennzeichnet, daß** die Verweildauer des Mahlguts in der Rührwerkskugelmühle 5 bis 60 Minuten, vorzugsweise 10 bis 45 Minuten, beträgt.

**7.** Verfahren gemäß einem oder mehreren der Ansprüche 1 bis 6, **dadurch gekennzeichnet, daß** man solche anionaktiven, kationaktiven und/oder nichtionogenen Tenside einsetzt, die bei der Destillation des Methylamins oder bei der Mahlung nicht schäumen.

**8.** Verfahren gemäß einem oder mehreren der Ansprüche 1 bis 7, **dadurch gekennzeichnet, daß** man als Pigmentdispergatoren solche der allgemeinen Formel (I)

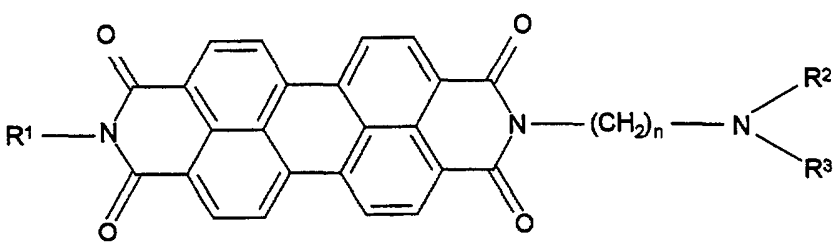

(I)

in welcher

$R^1$ ein Wasserstoffatom, eine Hydroxy- oder Aminogruppe, oder eine $C_1$-$C_{20}$-Alkylgruppe, die durch ein oder mehrere Chlor- oder Bromatome, eine Phenylgruppe, ein oder mehrere Cyan-, Hydroxy-, Carbamoyl-, $C_2$-$C_4$-Acyl- oder $C_1$-$C_4$-Alkoxygruppen substituiert sein kann oder perfluoriert oder teilfluoriert ist, und
$R^2$ und $R^3$ unabhängig voneinander ein Wasserstoffatom, eine substituierte oder unsubstituierte, oder teil- oder perfluorierte Alkylgruppe mit 1 bis 20 Kohlenstoffatomen, oder eine substituierte oder unsubstituierte, oder teil- oder perfluorierte Alkenylgruppe mit 2 bis 20 Kohlenstoffatomen. darstellen, wobei die Substituenten Hydroxy, Phenyl, Cyano, Chlor, Brom, $C_2$-$C_4$-Acyl oder $C_1$-$C_4$-Alkoxy sein und vorzugsweise 1 bis 4 an der Zahl sein können, oder $R^2$ und $R^3$ zusammen mit dem N-Atom einen gesättigten, ungesättigten oder aromatischen heterocyclischen Ring bilden, der gegebenenfalls ein weiteres Stickstoff-, Sauerstoff- oder Schwefelatom im Ring enthält; und
n eine Zahl von 1 bis 6 ist;

und/oder solche der allgemeinen Formel (II)

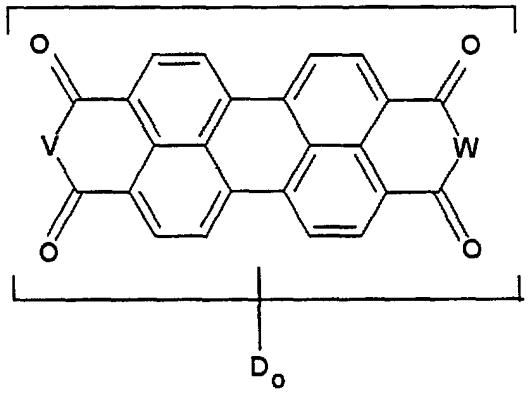

(II)

in welcher

V einen bivalenten Rest -O-, $>NR^4$ oder $>NR^5\text{-}Y^- X^+$ und
W den bivalenten Rest $>NR^5\text{-}Y^- X^+$ bedeutet,
D ein Chlor- oder Bromatom und sofern o > 1 ggf. eine Kombination davon darstellt, und o eine Zahl von 0 bis 4 ist;
$R^4$ für ein Wasserstoffatom, eine $C_1$-$C_{18}$-Alkylgruppe oder für eine Phenylgruppe steht, die unsubstituiert oder durch Halogen, $C_1$-$C_4$-Alkyl, $C_1$-$C_4$-Alkoxy oder Phenylazo ein- oder mehrfach substituiert sein kann,
$R^5$ für eine $C_1$-$C_{18}$-Alkylengruppe steht, die innerhalb der C-C-Kette durch ein Brückenglied aus der Reihe -O-, $-NR^6$-, -S-, Phenylen, -CO-, $-SO_2$- oder $-CR^7R^8$- oder eine chemisch sinnvolle Kombination davon ein- oder mehrfach unterbrochen sein kann und in dem $R^6$, $R^7$ und $R^8$ unabhängig voneinander jeweils ein Wasserstoffatom oder eine $C_1$-$C_4$-Alkylgruppe sind, die unsubstituiert oder durch einen heterocyclischen Rest substituiert sein kann, oder $R^5$ für eine Phenylengruppe steht, die unsubstituiert oder durch $C_1$-$C_4$-Alkyl oder $C_1$-$C_4$-Alkoxy ein- oder mehrfach substituiert sein kann,
$Y^-$ einen der anionischen Reste $-SO_3^-$ oder $-COO^-$ bedeutet, und
$X^+$ die Bedeutung $H^+$ oder das Äquivalent

$$\frac{M^{m+}}{m}$$

eines Metallkations aus der 1. bis 5. Hauptgruppe oder aus der 1. oder 2. oder der 4. bis 8. Nebengruppe des Periodensystems der chemischen Elemente bezeichnet, wobei m eine der Zahlen 1, 2 oder 3 ist, oder ein Ammoniumion $N^+R^9R^{10}R^{11}R^{12}$ definiert, wobei die Substituenten $R^9$, $R^{10}$, $R^{11}$ und $R^{12}$ am quartären N-Atom unabhängig voneinander jeweils ein Wasserstoffatom oder eine Gruppe aus der Reihe $C_2$-$C_{30}$-Alkyl, $C_2$-$C_{30}$-Alkenyl, $C_5$-$C_{30}$-Cycloalkyl, unsubstituiertes oder durch $C_1$-$C_8$-alkyliertes Phenyl oder eine (Poly)alkylenoxygruppe

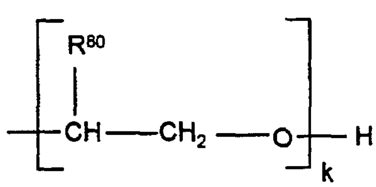

sind, in der $R^{80}$ für Wasserstoff oder $C_1$-$C_4$-Alkyl steht und k eine Zahl von 1 bis 30 ist;
und worin als $R^9$, $R^{10}$, $R^{11}$ und $R^{12}$ ausgewiesenes Alkyl, Alkenyl, Cycloalkyl, Phenyl oder Alkylphenyl durch Amino, Hydroxy und/oder Carboxy substituiert sein können; oder wobei die Substituenten $R^9$ und $R^{10}$ zusammen mit dem quartären N-Atom ein fünf- bis siebengliedriges gesättigtes Ringsystem, das ggf. noch weitere der Heteroatome O, S und/oder N enthält, bilden können,
oder wobei die Substituenten $R^9$, $R^{10}$ und $R^{11}$ zusammen mit dem quartären N-Atom ein fünf- bis siebengliedriges aromatisches Ringsystem, das ggf. noch weitere der Heteroatome O, S und/oder N enthält und an dem

ggf. zusätzliche Ringe ankondensiert sind, bilden können,

und/oder solche der allgemeinen Formel (III)

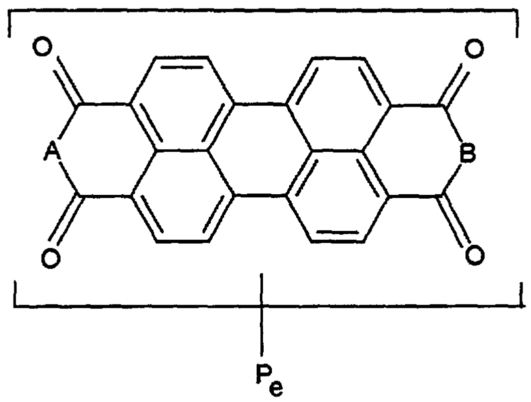

(III)

in welcher

A einen bivalenten Rest der Formel

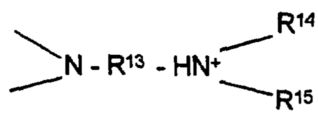

und
B einen bivalenten Rest der Formel

$$>N-R^{16}-SO_3^- \quad \text{oder} \quad >N-R^{16}-COO^-$$

bedeutet,
e einen Wert von 0 bis 8 hat, und, wenn e > 0 ist,
P ein Chlor- oder Bromatom und sofern e > 1 ist ggf. eine Kombination davon darstellt,
$R^{13}$ für eine $C_1$-$C_{12}$-Alkylengruppe, eine Aralkylengruppe oder eine Arylengruppe, steht,
$R^{14}$ und $R^{15}$ gleich oder verschieden sind und ein Wasserstoffatom, einen substituierten oder unsubstituierten $C_1$-$C_{20}$-Alkylrest, oder einen substituierten oder unsubstituierten $C_2$-$C_{20}$-Alkenylrest bedeuten, aber $R^{14}$ und $R^{15}$ nicht gleichzeitig Wasserstoff sind, oder
$R^{14}$ und $R^{15}$ zusammen mit dem angrenzenden N-Atom ein heterocyclisches Ringsystem bilden, das ggf. noch weitere der Heteroatome O, S und/oder N im Ring enthält und an das ggf. zusätzliche Ringe ankondensiert sind, und
$R^{16}$ eine geradkettige oder verzweigte $C_1$-$C_{12}$-Alkylengruppe darstellt; und/oder solche der allgemeinen Formel (IV)

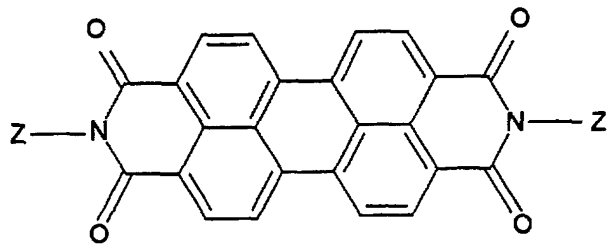

(IV)

in welcher Z die Bedeutung $Z^1$, $Z^2$ oder $Z^3$ hat, mit der Maßgabe, daß beide Z nicht gleichzeitig $Z^3$ sind, wobei $Z^1$ ein Rest der Formel (IVa) ist,

$$- [X- Y]_q-[X^1-Y^1]_r-[X^2-NH]_sH \qquad \text{(IVa)}$$

worin

X, $X^1$ und $X^2$ gleich oder verschieden sind und einen verzweigten oder unverzweigten $C_2$-$C_6$-Alkylenrest oder einen $C_5$-$C_7$-Cycloalkylenrest bedeuten, der durch 1 bis 4 $C_1$-$C_4$-Alkylreste, Hydroxyreste, Hydroxyalkylreste mit 1 bis 4 Kohlenstoffatomen und/oder durch 1 bis 2 weitere $C_5$-$C_7$-Cycloalkylreste substituiert sein kann;
Y und $Y^1$ gleich oder verschieden sind und eine NH-, -O-, N($C_1$-$C_6$-alkyl)-Gruppe, oder

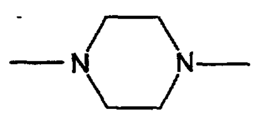

bedeuten;
q eine Zahl von 1 bis 6;
r und s unabhängig voneinander eine Zahl von 0 bis 6,
wobei r und s nicht gleichzeitig Null sind;
$Z^2$ ein Rest der Formel (IVb) ist,

$$-[X - O]_{q1} - [X^1 - O]_qH \qquad \text{(IVb)}$$

worin

q1 eine Zahl von 0 bis 6; und
$Z^3$ Wasserstoff, Hydroxy, Amino oder $C_1$-$C_8$-Alkyl ist, wobei die Alkylgruppe durch 1 bis 4 Substituenten aus der Gruppe Cl, Br, CN, OH, $C_6H_5$, Carbamoyl, $C_1$-$C_4$-Acyl, $C_1$-$C_4$-Alkoxy und $NR^2R^3$ substituiert sein kann, oder perfluoriert oder teilfluoriert ist; wobei
$R^2$ und $R^3$ unabhängig voneinander ein Wasserstoffatom, eine substituierte oder unsubstituierte, oder teil- oder perfluorierte Alkylgruppe mit 1 bis 20 Kohlenstoffatomen, oder eine substituierte oder unsubstituierte, oder teil- oder perfluorierte Alkenylgruppe mit 2 bis 20 Kohlenstoffatomen darstellen, wobei die Substituenten Hydroxy, Phenyl, Cyano, Chlor, Brom, $C_2$-$C_4$-Acyl oder $C_1$-$C_4$-Alkoxy sein können, oder $R^2$ und $R^3$ zusammen mit dem N-Atom einen gesättigten, ungesättigten oder aromatischen heterocyclischen Ring bilden, der gegebenenfalls ein weiteres Stickstoff-, Sauerstoff- oder Schwefelatom im Ring enthält.

**9.** Verfahren nach mindestens einem der Ansprüche 1 bis 8, **dadurch gekennzeichnet, daß** man als Tenside Fettsäuretauride, Harzseifen, Ammoniumsalze von Fettaminen oder ein Kondensationsprodukt von Cyanurchlorid, Taurin, N,N'-Diethylaminopropylamin und p-Phenylendiamin einsetzt.

**10.** Verfahren nach mindestens einem der Ansprüche 1 bis 9, **dadurch gekennzeichnet, daß** man insgesamt 1 bis

25 Gew.-%, bevorzugt 5 bis 15 Gew.-%, an Tensiden und/oder Pigmentdispergatoren, bezogen auf das Rohpigment zugibt.

## Claims

1. A process for preparing N,N'-dimethylperylene-3,4,9,10-tetracarboxylic diimide, utilizing the reaction of perylene-3,4,9,10-tetracarboxylic dianhydride with monomethyl-amine, which comprises employing at least 2 times the molar amount of monomethylamine per mole of perylene-3,4,9,10-tetracarboxylic dianhydride and at least 3 times the amount by weight of water based on the weight of the dianhydride, conducting the reaction at a temperature from 50 to 200°C, removing unreacted monomethylamine by distillation, subjecting the resulting crude pigment to wet grinding in a liquid, aqueous or aqueous-organic medium in a stirred ballmill which is operated with a power density of more than 1.5 kW per liter of milling space and a stirrer tip speed of more than 12 m/s under the action of grinding media having a diameter of less than or equal to 0.9 mm, and isolating the pigment obtained, with the proviso that at any desired point during the process, but no later than during wet grinding, one or more additives from the group consisting of pigment dispersants and surfactants are added.

2. The process as claimed in claim 1, wherein from 3 to 10 times the molar amount, preferably from 3 to 8 times the molar amount, of monomethylamine is employed per mole of perylene-3,4,9,10-tetracarboxylic dianhydride.

3. The process as claimed in claim 1 or 2, wherein the monomethylamine is added at from 0 to 80°C, preferably from 20 to 60°C.

4. The process as claimed in at least one of claims 1 to 3, wherein from 5 to 12 times, preferably from 6 to 9 times, the amount by weight of water, based on the weight of the dianhydride, is employed.

5. The process as claimed in at least one of claims 1 to 4, wherein the crude pigment in aqueous suspension is not isolated but passed directly to the wet grinding operation.

6. The process as claimed in one or more of claims 1 to 5, wherein the residence time of the millbase in the stirred ballmill is from 5 to 60 minutes, preferably from 10 to 45 minutes.

7. The process as claimed in one or more of claims 1 to 6, wherein anionic, cationic and/or nonionic surfactants which do not foam during the distillation of the methylamine or during grinding are employed.

8. The process as claimed in one or more of claims 1 to 7, wherein pigment dispersants employed are those of the formula (I)

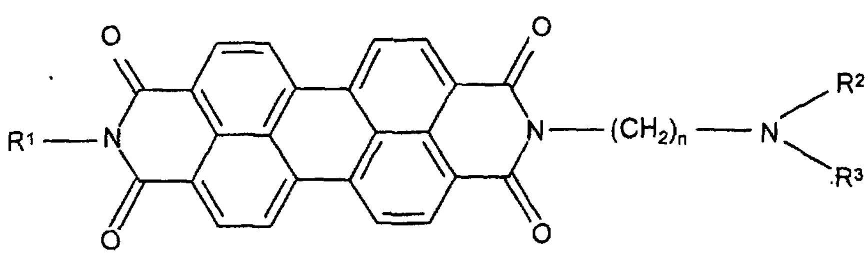

(I)

in which

$R^1$ is a hydrogen atom, a hydroxyl or amino group or a $C_1$-$C_{20}$-alkyl group which can be substituted by one or more chlorine or bromine atoms, a phenyl group, one or more cyano, hydroxyl, carbamoyl, $C_2$-$C_4$-acyl or $C_1$-$C_4$-alkoxy groups; or a $C_1$-$C_{20}$-alkyl group which is perfluorinated or partly fluorinated;
$R^2$ and $R^3$ independently of one another are a hydrogen atom, a substituted or unsubstituted, or perfluorinated or partly fluorinated alkyl group of 1 to 20 carbon atoms, or a substituted or unsubstituted, or perfluorinated or partly fluorinated alkenyl group of 2 to 20 carbon atoms, it being possible for the substituents to be hydroxyl, phenyl, cyano, chlorine, bromine, $C_2$-$C_4$-acyl or $C_1$-$C_4$-alkoxy and to be preferably 1 to 4 in number or $R^2$ and

$R^3$, together with the nitrogen atom, form a saturated, unsaturated or aromatic heterocyclic ring which if desired contains a further nitrogen, oxygen or sulfur atom in the ring; and
n is a number from 1 to 6;

and/or compounds having the formula (II)

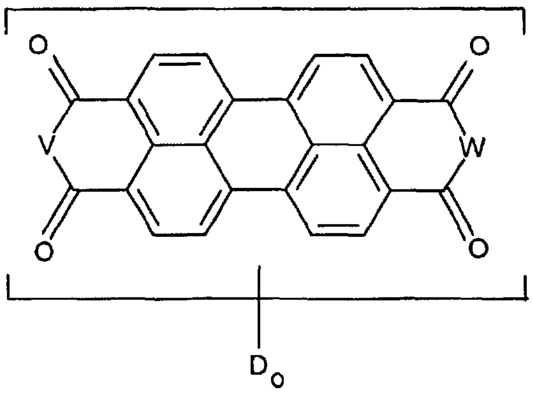

(II)

are employed in which

V is a bivalent radical -O-, >$NR^4$ or >$NR^5$-$Y^-$ $X^+$ and
W is the bivalent radical >$NR^5$-$Y^-$ $X^+$,
D is a chlorine or bromine atom and if o > 1, may represent a combination thereof, and o is a number from 0 to 4;
$R^4$ is a hydrogen atom or a $C_1$-$C_{18}$-alkyl group, or is a phenyl group which is unsubstituted or can be substituted one or more times, by halogen, $C_1$-$C_4$-alkyl, $C_1$-$C_4$-alkoxy, or phenylazo,
$R^5$ is a $C_1$-$C_{18}$-alkylene group which within the C-C chain can be interrupted one or more times, by a bridging link from the series consisting of -O-, -$NR^6$-, -S-, phenylene, -CO-, -$SO_2$- and -$CR^7R^8$- or any chemically possible combination thereof and in which $R^6$, $R^7$ and $R^8$ independently of one another are each a hydrogen atom or a $C_1$-$C_4$-alkyl group which can be unsubstituted or substituted by a heterocyclic radical, or $R^5$ is a phenylene group which can be unsubstituted or substituted one or more times by $C_1$-$C_4$-alkyl or by $C_1$-$C_4$-alkoxy,
$Y^-$ is one of the anionic radicals -$SO_3^-$ and -$COO^-$, and
$X^+$ is $H^+$ or the equivalent

$$\frac{M^{m+}}{m}$$

of a metal cation from main groups 1 to 5 or transition groups 1 or 2 or 4 to 8 of the Periodic Table of the Chemical Elements, m being 1, 2 or 3, or defines an ammonium ion $N^+R^9R^{10}R^{11}R^{12}$, the substituents $R^9$, $R^{10}$, $R^{11}$ and $R^{12}$ on the quaternary nitrogen atom being each independently of one another a hydrogen atom or a group from the series consisting of $C_1$-$C_{30}$-alkyl, $C_2$-$C_{30}$-alkenyl, $C_5$-$C_{30}$-cycloalkyl, unsubstituted or $C_1$-$C_8$-alkylated phenyl or a (poly)alkyleneoxy group

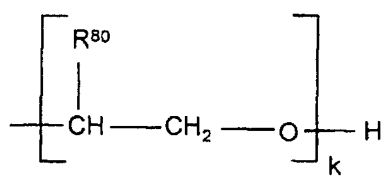

in which $R^{80}$ is hydrogen or $C_1$-$C_4$-alkyl and k is a number from 1 to 30;
and in which alkyl, alkenyl, cycloalkyl, phenyl or alkylphenyl identified as $R^9$, $R^{10}$, $R^{11}$ and $R^{12}$ can be substituted by amino, hydroxyl and/or carboxyl;
or where the substituents $R^9$ and $R^{10}$ together with the quaternary nitrogen atom, may form a five- to seven-membered saturated ring system which if desired includes further oxygen, sulfur and/or nitrogen heteroatoms, or where the substituents $R^9$, $R^{10}$ and $R^{11}$, together with the quaternary nitrogen atom, may form a 5- to 7-membered aromatic ring system which if desired includes further oxygen, sulfur and/or nitrogen heteroatoms and to which, if desired, additional rings are fused on, and/or compounds having the formula (III)

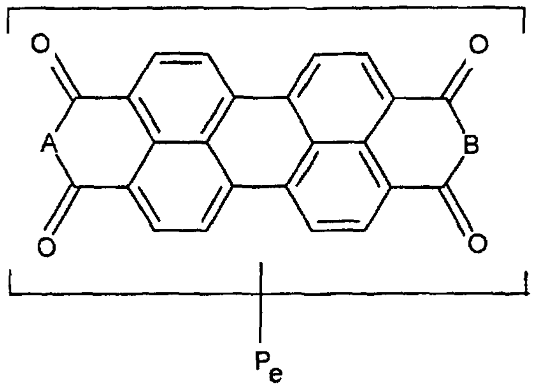

(III)

are employed in which

A is a bivalent radical of the formula

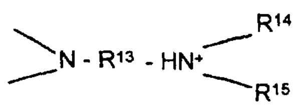

and
B is an bivalent radical of the formula

$>N-R^{16}-SO_3^-$ or $>N-R^{16}-COO^-$

e is a number from 0 to 8 and, if e > 0,
P is a chlorine or bromine atom, and if e > 1, P may represent a combination thereof,
$R^{13}$ is a $C_1$-$C_{12}$-alkylene group, an aralkylene group or an arylene group,
$R^{14}$ and $R^{15}$ are identical or different and are a hydrogen atom, a substituted or unsubstituted $C_1$-$C_{20}$-alkyl radical, or a substituted or unsubstituted $C_2$-$C_{20}$-alkenyl radical, but $R^{14}$ and $R^{15}$ are not simultaneously hydrogen, or
$R^{14}$ and $R^{15}$, together with the adjacent nitrogen atom, form a heterocyclic ring system which may contain yet more of the heteroatoms O, S and/or N in the ring and to which, if desired, additional rings are fused on, and
$R^{16}$ is a straight-chain or branched $C_1$-$C_{12}$-alkylene group;

and/or compounds having the formula (IV)

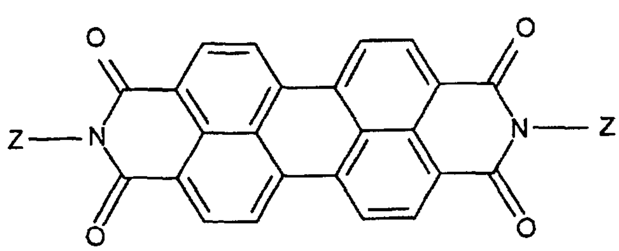

(IV)

are employed, in which Z has the definition $Z^1$, $Z^2$ or $Z^3$, with the proviso that the two Zs are not simultaneously $Z^3$, where
$Z^1$ is a radical of the formula (IVa)

$$- [X- Y]_q - [X^1 - Y^1]_r - [X^2 -NH]_s H \qquad \text{(IVa)}$$

in which

X, $X^1$ and $X^2$ are identical or different and are a branched or unbranched $C_2$-$C_6$-alkylene radical, or a $C_5$-$C_7$-cycloalkylene radical which can be substituted by from 1 to 4 $C_1$-$C_4$-alkyl radicals, hydroxyl radicals, hydroxyalkyl radicals of 1 to 4 carbon atoms and/or by 1 to 2 further $C_5$-$C_7$-cycloalkyl radicals;
Y and $Y^1$ are identical or different and are an NH-, -O- or N($C_1$-$C_6$-alkyl) group,
or

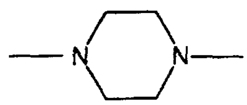

q is a number from 1 to 6,
r and s independently of one another are a number from 0 to 6, and r and s are not simultaneously zero;
$Z^2$ is a radical of the formula (IVb)

$$- [X - O]_{q1} - [X^1 - O]_q H \qquad \text{(IVb)}$$

in which

q1 is a number from 0 to 6; and
$Z^3$ is hydrogen, hydroxyl, amino or $C_1$-$C_8$-alkyl, it being possible for the alkyl group to be substituted by 1 to 4 substituents from the group consisting of Cl, Br, CN, OH, $C_6H_5$, carbamoyl, $C_1$-$C_4$-acyl, $C_1$-$C_4$-alkoxy and $NR^2R^3$, or is perfluorinated or partly fluorinated, where
$R^2$ and $R^3$ independently of one another are a hydrogen atom or a substituted or unsubstituted, or perfluorinated or partly fluorinated alkyl group of 1 to 20 carbon atoms, or a substituted or unsubstituted, or perfluorinated or partly fluorinated alkenyl group of 2 to 20 carbon atoms, it being possible for the substituents to be hydroxyl, phenyl, cyano, chlorine, bromine, $C_2$-$C_4$-acyl or $C_1$-$C_4$-alkoxy, or $R^2$ and $R^3$, together with the nitrogen atom, form a saturated, unsaturated or aromatic heterocyclic ring which if desired contains a further nitrogen, oxygen or sulfur atom in the ring.

**9.** The process as claimed in at least one of claims 1 to 8, wherein surfactants employed are fatty acid taurides, resin soaps, ammonium salts of fatty amines, or a condensation product of cyanuric chloride, taurine, N,N'-diethylaminopropylamine and p-phenylenediamine.

**10.** The process as claimed in at least one of claims 1 to 9, wherein a total of from 1 to 25% by weight, preferably from 5 to 15% by weight, of surfactants and/or pigment dispersants is added, based on the crude pigment.

**Revendications**

**1.** Procédé pour la préparation du N,N'-diméthylpérylène-3,4,9,10-pérylènetétracarboxydiimide en utilisant la réaction du dianhydride de l'acide pérylène-3,4,9,10-pérylènetétracarboxylique avec la monométhylamine, **caractérisé en ce qu'**on utilise par mole de dianhydride de l'acide pérylène-3,4,9,10-pérylène-tétracarboxylique une quantité au moins 2 fois molaire de monométhylamine et au moins 3 fois molaire d'eau, par rapport au poids du dianhydride, on met en oeuvre la réaction à une température de 50 à 200°C, on chasse par distillation la monométhylamine n'ayant pas réagi, on broie par voie humide le pigment brut obtenu dans un milieu liquide, aqueux ou aqueux organique, sur un broyeur à microbilles, qu'on exploite avec une puissance volumique supérieure à 1,5 kW par

litre de chambre de broyage et une vitesse périphérique de broyeur supérieure à 12 m/s, sous l'effet de corps broyants ayant un diamètre égal ou inférieur à 0,9 mm, et on isole le pigment obtenu ; à condition qu'on ajoute à à un moment arbitraire du procédé, mais au plus tard au cours du broyage par voie humide, une ou plusieurs matières d'addition prises dans le groupe des dispersants de pigment et des agents de surface.

**2.** Procédé selon la revendication 1, **caractérisé en ce qu'**on utilise par mole de dianhydride de l'acide pérylène-3,4,9,10-pérylène-tétracarboxylique la quantité 3 à 10 fois molaire, de préférence 3 à 8 fois molaire, de monométhylamine.

**3.** Procédé selon la revendication 1 ou 2, **caractérisé en ce qu'**on ajoute la monométhylamine à une température de 0 à 80°C, de préférence de 20 à 60°C.

**4.** Procédé selon une des revendications 1 à 3, **caractérisé en ce qu'**on utilise une quantité 5 à 12 fois, de préférence 6 à 9 fois en poids d'eau, par rapport au dianhydride.

**5.** Procédé selon une des revendications 1 à 4, **caractérisé en ce qu'**on apporte le pigment brut en suspension aqueuse sans isolement intermédiaire, au broyage par voie humide.

**6.** Procédé selon une des revendications 1 à 5, **caractérisé en ce que** la durée de séjour de la matière à broyer dans le broyeur à microbilles est de 5 à 60 minutes, de préférence de 10 à 45 minutes.

**7.** Procédé selon une ou plusieurs des revendications 1 à 6, **caractérisé en ce qu'**on utilise de tels agents de surface anioniques, cationiques et/ou non ionogènes, qui ne moussent pas au cours de la distillation de la méthylamine ou au cours du broyage.

**8.** Procédé selon une ou plusieurs des revendications 1 à 7, **caractérisé en ce qu'**on utilise en tant que dispersants de pigments ceux de formule générale (I)

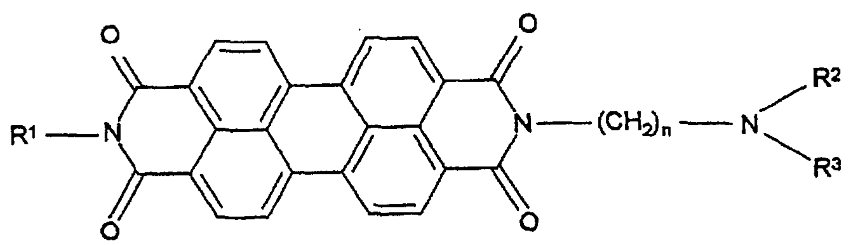

(I)

dans laquelle

$R^1$ représente un atome d'hydrogène, un groupe hydroxy ou amino, ou un groupe alkyle en $C_1$-$C_{20}$, qui peut être substitué par un ou plusieurs atomes de chlore ou de brome, un groupe phényle, un ou plusieurs groupes cyano, hydroxy, carbamoyle, acyle en $C_2$-$C_4$ ou alkoxy en $C_1$-$C_4$, ou peut être perfluoré ou partiellement fluoré ; et
$R^2$ et $R^3$ représentent, indépendamment l'un de l'autre, un atome d'hydrogène, un groupe alkyle présentant 1 à 20 atomes de carbone substitué ou non substitué, partiellement fluoré ou perfluoré, ou un groupe alcényle présentant 2 à 20 atomes de carbone substitué ou non substitué, partiellement fluoré ou perfluoré, les substituants pouvant être hydroxy, phényle, cyano, chloro, bromo, acyle en $C_2$-$C_4$ ou alkoxy en $C_1$-$C_4$ et de préférence leur nombre peut être de 1 à 4, ou $R^2$ et $R^3$ ensemble avec l'atome de N, forment un cycle hétérocyclique saturé, insaturé ou aromatique, qui contient éventuellement un autre atome d'azote, d'oxygène ou de soufre dans le cycle ; et
n va de 1 à 6 ;

et/ou ceux de formule générale (II)

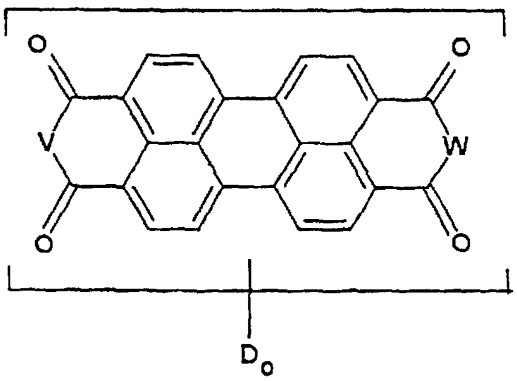

(II)

dans laquelle

V représente un reste bivalent -O-, >$NR^4$ ou >$NR^5$-$Y^-X^+$ et
W représente le reste bivalent >$NR^5$-$Y^-X^+$,
D représente un atome de chlore ou de brome et dans la mesure où o>1, éventuellement une de leurs combinaisons et o va de 0 à 4 ;
$R^4$ représente un atome d'hydrogène, un groupe alkyle en $C_1$-$C_{18}$ ou un groupe phényle, qui est non substitué ou peut être substitué une ou plusieurs fois par des atomes d'halogènes, des groupes alkyle en $C_1$-$C_4$, alkoxy en $C_1$-$C_4$ ou phénylazo,
$R^5$ représente un groupe alkylène en $C_1$-$C_{18}$ qui peut être interrompu une ou plusieurs fois à l'intérieur de la chaîne C-C par un élément de pontage pris dans le groupe -O-, -$NR^6$-, -S-, phénylène, -CO-, -$SO_2$- ou $CR^7R^8$- ou une combinaison chimique pertinente, et dans lequel les constituants $R^6$, $R^7$ et $R^8$ représentent chacun, indépendamment l'un de l'autre, un atome d'hydrogène ou un groupe alkyle en $C_1$-$C_4$, qui est non substitué ou peut être substitué par un reste hétérocyclique, ou $R^5$ représente un groupe phénylène qui est non substitué ou peut être substitué une ou plusieurs fois par un groupe alkyle en $C_1$-$C_4$ ou alkoxy en $C_1$-$C_4$,
$Y^-$ représente le reste anionique -$SO_3^-$ ou -$COO^-$, et
$X^+$ possède la signification de $H^+$ ou représente l'équivalent

$$\frac{M^{m+}}{m}$$

d'un cation métallique pris dans le groupe principal 1 à 5 ou: le sous-groupe 1 ou 2 ou 4 à 8 de la Classification périodique des éléments chimiques, où m vaut 1, 2 ou 3, ou un ion ammonium $N^+R^9R^{10}R^{11}R^{12}$, les substituants $R^9$, $R^{10}$, $R^{11}$ et $R^{12}$ sur l'atome de N quaternaire représentant chacun, indépendamment l'un de l'autre, un atome d'hydrogène ou un groupe de la série alkyle en $C_1$-$C_{30}$, alcényle en $C_2$-$C_{30}$, cycloalkyle en $C_5$-$C_{30}$ non substitué ou substitué par un substituant phényle alkylé en $C_1$-$C_8$ ou un groupe (poly)oxyalkylène

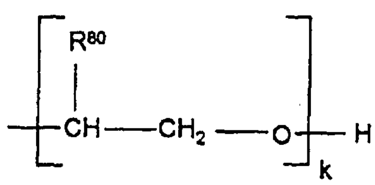

où $R^{80}$ représente un atome d'hydrogène ou un groupe alkyle en $C_1$-$C_4$ et k est un nombre de 1 à 30 ;
et où les groupes alkyle, alcényle, cycloalkyle, phényle ou alkylphényle précités en tant que $R^9$, $R^{10}$, $R^{11}$ et $R^{12}$ peuvent être substitués par des substituants amino, hydroxy et/ou carboxy ; ou les substituants $R^9$ et $R^{10}$ pouvant former, conjointement avec l'atome de N quaternaire, un système cyclique saturé de 5 à 7 chaînons, qui peut éventuellement contenir encore d'autres hétéroatomes O, S et/ou N ;
ou les substituants $R^9$, $R^{10}$ et $R^{11}$ pouvant former, conjointement avec l'atome de N quaternaire, un système cyclique aromatique de 5 à 7 chaînons qui peut éventuellement contenir encore d'autres hétéroatomes O, S et/ou N et sur lequel sont éventuellement condensés d'autres cycles ;

et/ou ceux de formule générale (III)

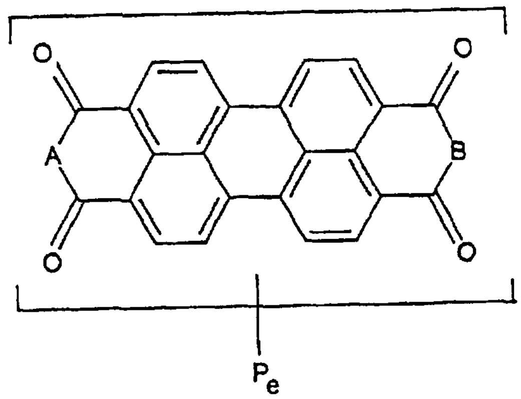

(III)

dans laquelle

A représente un reste bivalent cationique de formule

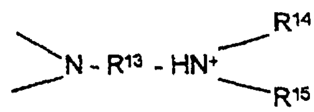

et

B représente un reste bivalent anionique de formule

$>N-R^{16}-SO_3^-$ ou $>N-R^{16}-COO^-$

e est une valeur de 0 à 8, et lorsque e>0,

P représente un atome de chlore ou de brome et dans la mesure où e>1, éventuellement une de leurs combinaisons,

$R^{13}$ représente un groupe alkylène en $C_1$-$C_{12}$, un groupe aralkylène ou un groupe arylène,

$R^{14}$ et $R^{15}$ sont identiques ou différents et représentent un atome d'hydrogène, un reste alkyle en $C_1$-$C_{20}$ substitué ou non substitué, ou un reste alcényle en $C_2$-$C_{20}$ substitué ou non substitué, mais $R^{14}$ et $R^{15}$ ne sont pas simultanément un atome d'hydrogène, ou

$R^{14}$ et $R^{15}$ forment, conjointement avec l'atome de N adjacent, un système hétérocyclique, qui éventuellement contient d'autres hétéroatomes O, S et/ou N dans le cycle et sur lequel sont éventuellement condensés d'autres cycles, et

$R^{16}$ représente un groupe alkylène en $C_1$-$C_{12}$ linéaire ou ramifié ;

et/ou ceux de formule générale (IV)

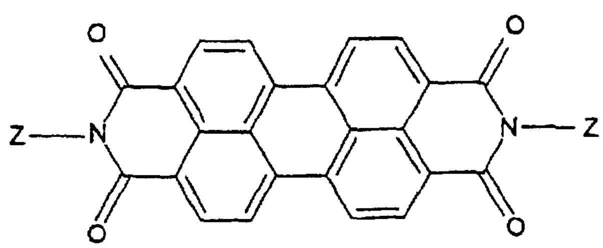

(IV)

dans laquelle Z représente $Z^1$, $Z^2$ ou $Z^3$, à condition que les deux Z ne soient pas simultanément $Z^3$, où

$Z^1$ représente un reste de formule (IVa),

$$-[X\text{-}Y]_q\text{-}[X^1\text{-}Y^1]_r\text{-}[X^2\text{-}NH]_sH \qquad \text{(IVa)}$$

où

X, $X^1$ et $X^2$ sont identiques ou différents et représentent un reste alkylène en $C_2$-$C_6$ linéaire ou ramifié ou un reste cycloalkylène en $C_5$-$C_7$, qui peut être substitué par 1 à 4 restes alkyle en $C_1$-$C_4$, hydroxy, hydroxyalkyle ayant 1 à 4 atomes de carbone et/ou par 1 à 2 autres restes cycloalkyle en $C_5$-$C_7$ ;
Y et $Y^1$ sont identiques ou différents et représentent un groupe NH-, -O-, N(alkyle en $C_1$-$C_6$),

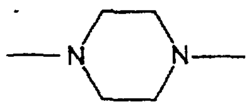

q est un nombre de 1 à 6 ;
r et s sont, indépendamment l'un de l'autre, un nombre de 0 à 6, r et s n'étant pas simultanément zéro ;
$Z^2$ représente un reste de formule (IVb),

$$-[X\text{-}O]_{q1}\text{-}[X^1\text{-}O]_qH \qquad \text{(IVb)}$$

q1 est un nombre de 0 à 6 ; et
$Z^3$ représente un atome d'hydrogène, des groupes hydroxy, amino ou alkyle en $C_1$-$C_8$, le groupe alkyle pouvant être substitué par 1 à 4 substituants pris dans le groupe des substituants Cl, Br, CN, OH, $C_6H_5$, carbamoyle, acyle en $C_1$-$C_4$ ou alkoxy en $C_1$-$C_4$ et $NR^2R^3$, ou est perfluoré ou partiellement fluoré ; où
$R^2$ et $R^3$ représentent, indépendamment l'un de l'autre, un atome d'hydrogène, un groupe alkyle présentant 1 à 20 atomes de carbone, substitué ou non substitué, ou partiellement fluoré ou perfluoré, ou un groupe alcényle présentant 2 à 20 atomes de carbone non substitué ou substitué, ou partiellement fluoré ou perfluoré, les substituants pouvant être hydroxy, phényle, cyano, chloro, bromo, acyle en $C_2$-$C_4$ ou alkoxy en $C_1$-$C_4$, ou $R^2$ et $R^3$ forment, conjointement avec l'atome de N, un hétérocycle saturé, insaturé ou aromatique, qui contient éventuellement un autre atome d'azote, d'oxygène ou de soufre.

**9.** Procédé selon au moins une des revendications 1 à 8, **caractérisé en ce qu'**on utilise comme agents de surface des taurides d'acides gras, des savons résineux, des sels d'ammonium d'amines grasses ou un produit de condensation de chlorure de cyanuryle, de taurine, de N,N'-diéthylaminopropylamine et de p-phénylènediamine.

**10.** Procédé selon au moins une des revendications 1 à 9, **caractérisé en ce qu'**on ajoute au total de 1 à 25 % en masse, de préférence de 5 à 15 % en masse, d'agents de surface et/ou dispersant de pigments, par rapport au pigment brut.